(19) Europäisches Patentamt
European Patent Office
Office européen des brevets

(11) Veröffentlichungsnummer: **0 277 916 B1**

(12) **EUROPÄISCHE PATENTSCHRIFT**

(45) Veröffentlichungstag der Patentschrift: **25.05.94**

(21) Anmeldenummer: **88810049.2**

(22) Anmeldetag: **29.01.88**

(51) Int. Cl.⁵: **C07D 493/22**, A61K 31/35, A01N 43/90, C12P 17/08, //(C07D493/22,313:00,311:00, 311:00,307:00),(C07D493/22, 313:00,311:00,311:00,307:00, 303:00)

# (54) Mikrobielles Verfahren zur Herstellung von Milbemycin-Derivaten.

(30) Priorität: **04.02.87 CH 395/87**

(43) Veröffentlichungstag der Anmeldung: **10.08.88 Patentblatt 88/32**

(45) Bekanntmachung des Hinweises auf die Patenterteilung: **25.05.94 Patentblatt 94/21**

(84) Benannte Vertragsstaaten: **AT BE CH DE ES FR GB GR IT LI LU NL SE**

(56) Entgegenhaltungen:
**EP-A- 0 110 667**
**EP-A- 0 184 989**

(73) Patentinhaber: **CIBA-GEIGY AG**
**Klybeckstrasse 141**
**CH-4002 Basel(CH)**

(72) Erfinder: **Ramos, Gerado, Dr.**
**General Guisan-Strasse 35**
**CH-4144 Arlesheim(CH)**

Erfinder: **Ghisalba, Oreste, Dr.**
**Eschenweg 3**
**CH-4153 Reinach(CH)**
Erfinder: **Schär, Hans-Peter, Dr.**
**Eggfluhweg 11**
**CH-4147 Aesch(CH)**
Erfinder: **Frei, Bruno, Dr.**
**Bündtenstrasse 16**
**CH-4410 Liestal(CH)**
Erfinder: **Maienfisch, Peter, Dr.**
**Traugott Meyer-Strasse 5**
**CH-4147 Aesch(CH)**
Erfinder: **O'Sullivan, Anthony Cornelius, Dr.**
**Habsburgerstrasse 38**
**CH-4055 Basel(CH)**

EP 0 277 916 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein einstufiges, mikrobielles Verfahren zur Herstellung von 13$\beta$-Hydroxi- oder 14,15-Epoxi-Milbemycinen der nachfolgenden Formeln I und II

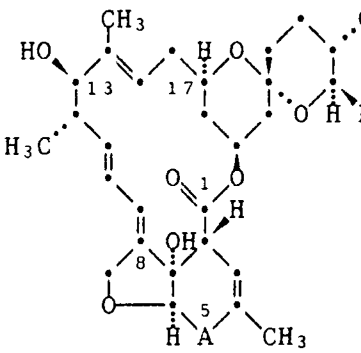 (I); 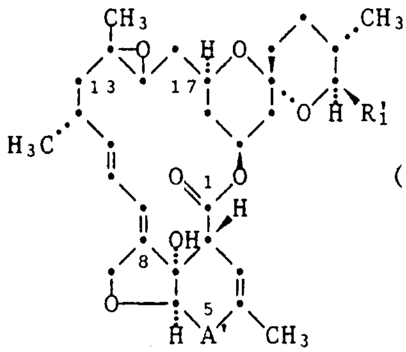 (II)

worin
$R_1$ und $R_1'$ für Methyl, Ethyl, Isopropyl oder sek.-Butyl oder für die Gruppe

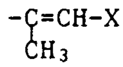

stehen, worin X Methyl, Ethyl oder Isopropyl bedeutet und
A und A' für die Gruppe

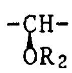 oder 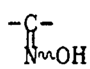

stehen, worin
$R_2$ Wasserstoff, $-C(O)-CH_2O-C(O)-CH_2CH_2-COOH$ oder $-C(O)-CH_2CH_2-COOH$ bedeutet, oder von Gemischen von Verbindungen der Formeln I und II, das sich dadurch kennzeichnet, dass
man ein in einer flüssigen Phase gelöstes Milbemycin der Fomel III

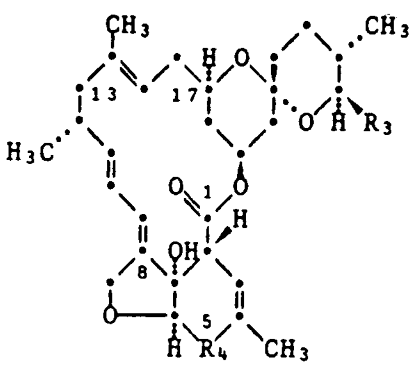 (III)

worin $R_3$ für $R_1$ oder $R_1'$ und $R_4$ für A oder A' steht und $R_1$, $R_1'$, A und A' die unter den Formeln I und II angegebenen Bedeutungen haben mit einem zur 13$\beta$-Hydroxilierung oder 14,15-Epoxidierung oder zu beiden Reaktionen befähigten Biokatalysator für einen Zeitraum in Kontakt bringt, der für die Durchführung der 13$\beta$-Hydroxilierungs-oder 14,15-Epoxidierungsreaktion oder beider Reaktionen ausreicht.

Ganz besonders bevorzugt im Rahmen dieser Erfindung ist ein Verfahren zur Herstellung von Verbindungen der nachfolgenden Formeln I und II

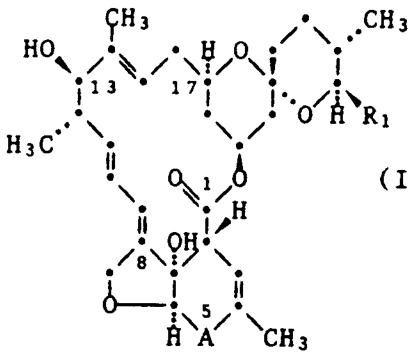
(I);

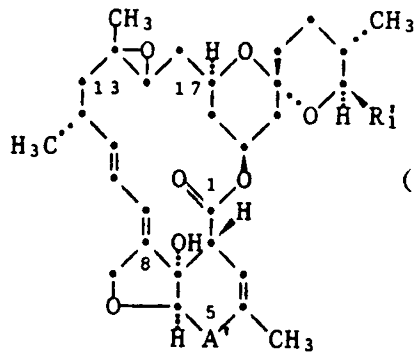
(II)

worin
$R_1$ und $R_1'$ für Methyl, Ethyl, Isopropyl oder sek.-Butyl und
A und A' für die Gruppe

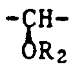 oder 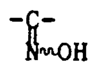

stehen, worin
$R_2$ Wasserstoff, -C(O)-$CH_2$O-C(O)-$CH_2CH_2$-COOH oder -C(O)-$CH_2CH_2$-COOH bedeutet, dadurch gekennzeichnet, dass
man ein in einer flüssigen Phase gelöstes Milbemycin der Formel III,

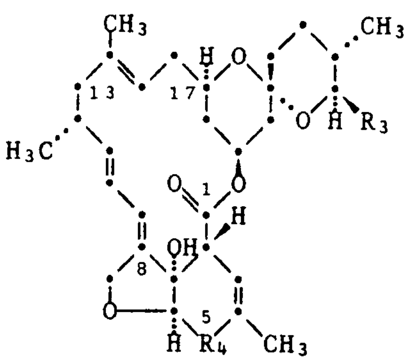
(III)

worin
$R_3$ für Methyl, Ethyl, Isopropyl oder sek.-Butyl und
$R_4$ für die Gruppe

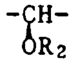 oder 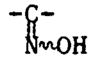

steht, worin
$R_2$ Wasserstoff, -C(O)-$CH_2$O-C(O)-$CH_2CH_2$-COOH oder -C(O)-$CH_2CH_2$-COOH bedeutet mit einem zur 13β-Hydroxilierung oder 14,15-Epoxidierung oder zu beiden Reaktionen befähigten Biokatalysator für einen Zeitraum in Kontakt bringt, der für die Durchführung der 13β-Hydroxilierungs-oder 14,15-Epoxidierungsreaktion oder beider Reaktionen ausreicht.

Verbindungen der Formeln I und II, worin $R_1$ und $R_1'$ für fie Gruppe

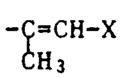

stehen und die Reste X, A, A′ und $R_2$ die in Formeln I und II angegebenen Bedeutungen haben, repräsentieren solche 23-Deoxy-Abkömmlinge der natürlichen Antibiotika S541, die eine 13β-Hydroxi- oder eine 14,15-Epoxigruppe enthalten und in 5-Position entweder eine freie OH-Gruppe oder eine Oxim-Gruppierung tragen.

Im allgemeinen werden bei diesem mikrobiellen Verfahren beide Produkte (I) und (II) gebildet, es entsteht jedoch (I) in der Regel im Ueberschuss, wobei man die Produkte (I) und (II) gewöhnlich im Verhältnis von ca. 4:1 erhält.

Die beiden so erhaltenen Reaktionsprodukte der Formeln I und II lassen sich ohne grossen technischen Aufwand durch übliche Trennungsmethoden, z.B. durch fraktionierte Kristallisation oder durch Chromatographie, voneinander trennen. Unter Chromatographie werden z.B. Säulen-, Dickschicht- oder Dünnschicht-Chromatographie an mineralischen Trägermaterialien wie Silicagel oder an organischen Austauscherharzen verstanden.

Falls nur Verbindungen der Formel I (13β-Hydroximilbemycine) im Vordergrund des Interesses stehen, so ist die Auftrennung von (I) und (II) nicht erforderlich, da die 14,15-Epoxide der Formel II gemäss EP-0,180,539 in die 13β-Hydroximilbemycine der Formel I überführt werden können.

Unter dem Begriff Mikroorganismus oder aktive Bestandteile davon werden im Rahmen der vorliegenden Erfindung verstanden:

a) der lebende Mikroorganismus in Form vegetativer Zellen,

b) der tote Mikroorganismus, vorzugsweise in einer aufgeschlossenen Form, d.h. mit mechanisch oder chemisch zertrümmerter oder entfernter Zellwand/-membran,

c) Rohextrakte der Zellinhaltsstoffe des besagten Mikroorganismus,

d) die Enzyme, die Verbindungen der Formel III in Verbindungen der Formel II und/oder I überführen und

e) die Sporen des besagten Mikroorganismus.

Die unter a) bis e) genannten aktiven Systeme, die erfindungsgemäss einsetzbar sind, sollen hier und im folgenden einfachheitshalber zu dem Begriff Biokatalysatoren zusammengefasst und dieser Begriff stellvertretend für jedes dieser Systeme verwendet werden.

Für das erfindungsgemässe Verfahren geeignete Mikroorganismen sind besonders unter den Bakterien und höheren Pilzen zu finden. Bei den Bakterien sind in erster Linie Vertreter der Actinomyceten, insbesondere der Gattung Streptomyces zu erwähnen; als besonders geeignet für die stereospezifische Einführung einer Hydroxigruppe in die 13β-Position und/oder für eine 14,15-Epoxidierung der Verbindungen der Formel III haben sich die Stämme Streptomyces diastatochromogenes ATCC 31561, vor allem aber Streptomyces violascens ATCC 31560 erwiesen.

Unter den höheren Pilzen sind in erster Linie Vertreter der Fungi imperfecti, insbesondere Vertreter aus der Gruppe der Moniliales geeignet. Ganz besonders gute Ergebnisse im Hinblick auf die Produkte der Formel I und II lassen sich bei Verwendung von Vertretern aus der Gattung Aspergillus erzielen, insbesondere mit dem Stamm Aspergillus niger sp. KS-101 ATCC 20567.

Die im Rahmen dieser Erfindung verwendeten Mikroorganismen, i.e. die Streptomyces-Stämme Streptomyces diastatochromogenes ATCC 31561 und Streptomyces violascens ATCC 31560 sowie der Aspergillus-Stamm Aspergillus niger sp. KS-101 ATCC 20567 sind in dem US-Patent Nr. 4,226,941 beschrieben.

Diese Beschreibung, welche die Spalten 3 bis 6 des besagten US-Patentes umfasst, ist in Form einer Referenz ein Bestandteil der vorliegenden Erfindung.

Die 13β-Hydroximilbemycine der Formel I mit einer freien oder acyclierten 5-Hydroxigruppe sind als ausserordentlich aktive Wirksubstanzen gegen Ekto- und Endoparasiten am Nutztier aus der EP-0,180,539 bekannt. Bei dem bekannten Verfahren (EP-0,180,539) zur Herstellung der Verbindungen der Formel I geht man wie bei dem erfindungsgemässen, mikrobiellen Verfahren von den Verbindungen der Formel III aus. Dabei werden die Verbindungen der Formel III in einer ersten Stufe mit Persäuren in die 14,15-Epoxide der Formel II überführt:

1. Stufe

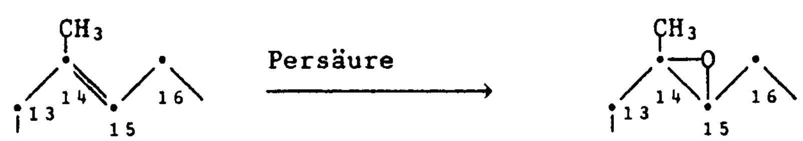


(III) (II)

und die 14,15-Epoxide der Formel II anschliessend mit Hilfe eines speziellen Komplexreagenz zu 15-Hydroxi-Verbindungen der Formel IV umgesetzt:

2. Stufe

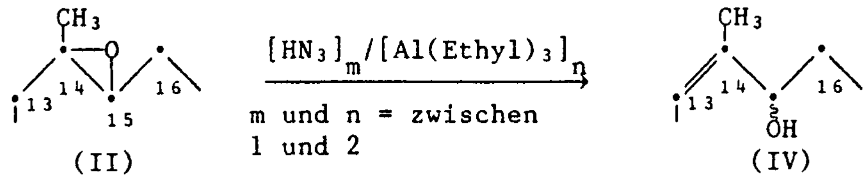


(II) (IV)

In einer weiteren Stufe werden dann die 15-Hydroxi-Verbindungen der Formel IV mit Chromat-, Halochromat- oder Dichromat-Ionen, insbesondere mit Pyridiniumdichromat umgesetzt:

3. Stufe

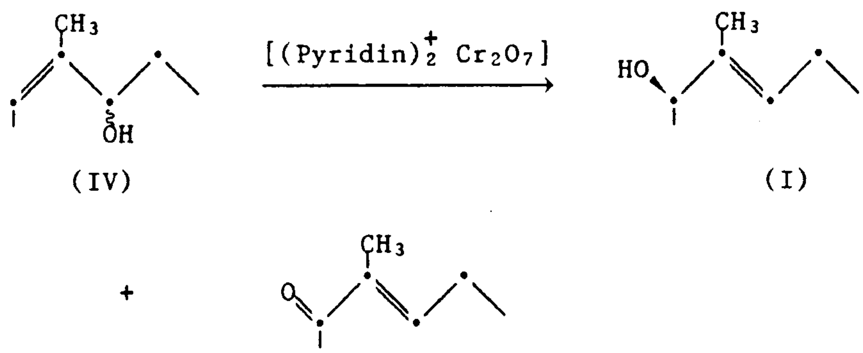


(IV) (I)

+

(V)

wobei ausser den erwünschten 13-Hydroxi-milbemycinen der Formel I auch 13-Oxo-Verbindungen entstehen, die abgetrennt werden müssen.

Das erfindungsgemässe, mikrobielle Hydroxilierungs-Verfahren hat gegenüber dem vorbekannten Verfahren den entscheidenden Vorteil, dass es nur eine Stüfe umfasst, zu höheren Gesamtausbeuten führt und weniger umweltbelastend ist, da weniger Chemikalien eingesetzt werden müssen, und als Nebenprodukt lediglich Biomasse entsteht.

Das beim erfindungsgemässen mikrobiellen Verfahren entstehende 14,15-Epoxid ist als wertvoller Baustein für die Herstellung unterschiedlich substituierter Milbemycine bekannt, so z.B. aus folgenden Europäischen Offenlegungsschriften: EP-0,180,539, EP-0,147,852, EP-0,184,989 und EP-0,184,173.

Die Verbindungen der Formeln I und II, worin A und A' für die Oximgruppe stehen, sind neu und stellen ihrerseits hochaktive Ekto- und Endo-parasitizide bzw. Zwischenprodukte zur Herstellung hochaktiver Ekto- und Endoparasitizide dar.

Folgende Untergruppen von Verbindungen der Formel I und II zeigen eine ausgeprägte parasitizide und insektizide Wirkung:

Gruppe Ia, IIa: Verbindungen der Formeln I und II, wobei $R_1$ und $R_1'$ für Methyl, Ethyl, Isopropyl oder sek.Butyl oder für die Gruppe

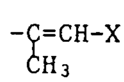

steht, worin X Methyl, Ethyl oder Isopropyl bedeutet und A und A' für die Gruppe

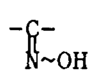

steht.

Gruppe Ib, IIb: Verbindungen der Formel I und II, wobei $R_1$ und $R_1'$ für Methyl, Ethyl, Isopropyl oder sek.Butyl und A und A' für die Gruppe

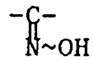

steht.

Gruppe Ic, IIc: Verbindungen der Formel I und II, wobei $R_1$ und $R_1'$ für Methyl oder Ethyl und A und A' für die Gruppe

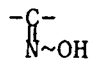

steht.

Insbesondere zu nennen sind in diesem Zusammenhang die im folgenden aufgeführten Einzelverbindungen:

14,15 Epoxi-5-oximino-milbemycin $A_4$
14,15 Epoxi-5-oximino-milbemycin $A_3$
14,15 Epoxi-5-oximino-milbemycin D

Milbemycine sind als hochaktive Ekto- und Endoparasitizide aus der Literatur bekannt, so z.B. diejenigen der nachfolgenden Formel (M):

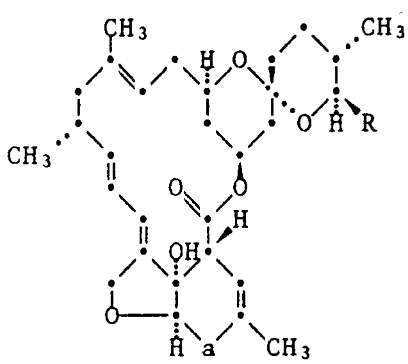

(M)

R = $CH_3$, a = 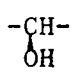 = Milbemycin $A_3$ aus US-PS 3,950,360

R = $C_2H_5$, a = 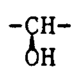 = Milbemycin $A_4$ aus US-PS 3,950,360

R = iso$C_3H_7$, a = 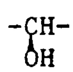 = Milbemycin D aus US-PS 4,346,171

R = sek.$C_4H_9$, a = 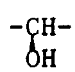 = 13-Desoxi-22,23-dihydro-C-076-Bla-aglycon, aus US-PS 4,173,571.

R = $CH_3$, a = 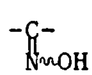 = 5-Oxim-Milbemycin $A_3$ aus US-PS 4,547,520

R = $C_2H_5$, a = 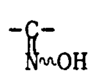 = 5-Oxim-Milbemycin $A_4$ aus US-PS 4,547,520

R = iso$C_3H_7$, a = 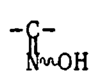 = 5-Oxim-Milbemycin $A_4$ aus US-PS 4,547,520

Verbindungen, worin R sek.Butyl darstellt, sollen hier und im folgenden gleichfalls zu den Milbemycin-Derivaten gerechnet werden, obwohl sie nach der üblichen Systematik von Avermectin-Derivaten abgeleitet

sind. Avermectin-Aglykone (mit einer OH-Gruppe in 13-Position) lassen sich jedoch gemäss US-PS 4,173,571 in Milbemycin-Homologe überführen.

Die Konstitution von natürlichen Antibiotika S541 ist aus der DE-OS-35 32 794 bekannt und sieht wie folgt aus:

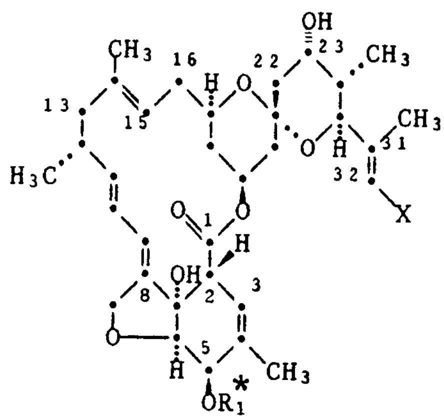

(Antibiotika S541)

| | | |
|---|---|---|
| Faktor A | X = iso$C_3H_7$ | $R_1^*$ = H |
| Faktor B | X = $CH_3$ | $R_1^*$ = $CH_3$ |
| Faktor C | X = $CH_3$ | $R_1^*$ = H |
| Faktor D | X = $C_2H_5$ | $R_1^*$ = H |
| Faktor E | X = $C_2H_5$ | $R_1^*$ = $CH_3$ |
| Faktor F | X = iso$C_3H_7$ | $R_1^*$ = $CH_3$ |

Je nach Faktor werden im folgenden zur Vereinfachung der Benennung die Abkömmlinge von Antibiotikum S541 als Derivate von S541A, S541B, S541C, S541D, S541E oder S541F klassifiziert.

Verbindungen der Formel III, worin $R_3$ für die Gruppe

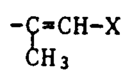

steht, wobei die Reste X und $R_4$ die für Formel III angegebene Bedeutung haben und die als Ausgangsprodukte in dem erfindungsgemässen Verfahren verwendet werden können, lassen sich auf an sich bekannte Weise aus den natürlichen Antibiotika S541 herstellen.

Die in 23-Position befindliche Hydroxygruppe in den Antibiotika S541 lässt sich analog zu der in US-PS 4,328,335 bekanntgewordenen Methode entfernen und die Antibiotika S541 lassen sich so in die entsprechenden 23-Deoxi-Derivate überführen. Dabei müssen diejenigen Verbindungen mit einer freien 5-OH-Gruppe ($R_1^*$ = H) zunächst selektiv geschützt werden durch Reaktion mit einem der nachstehend angegebenen Silylierungsreagenzien Y-Si($R_5$)($R_6$)($R_7$) bzw. mit tert.-Butyldimethylsilyloxiacetylchlorid. Die Umsetzung dieser geschützten Verbindungen, in denen $R_1^*$ durch Si($R_5$)($R_6$)($R_7$) bzw. C(=0)$CH_2$OSi$(CH_3)_2$t-$C_4H_8$ ersetzt und das 23-C-Atom durch OH substituiert ist, mit p-Methylphenyl-chlorthionoformiat ergibt Derivate der Antibiotika S541, welche in Position 23 durch -O-C(=S)-$OC_6H_4$-$CH_3$-p substituiert sind. Diese 23-O-(4-Methylphenoxi)-thiocarbonyl-derivate von Antibiotika S541 werden dann als Ausgangsmaterialien für die Reduktion mit Tributylzinnhydrid in Toluol in Gegenwart von Azobisisobutyronitril bei 80-120 °C zu den entsprechenden 23-Deoxi-Derivaten (Position 23 substituiert) eingesetzt.

Zur Silylierung verwendet man zweckmässigerweise ein Silan der Formel Y-Si($R_5$)($R_6$)($R_7$), worin $R_5$, $R_6$ und $R_7$ vorzugsweise unabhängig voneinander für $C_1$-$C_4$-Alkyl, Benzyl oder Phenyl stehen und der Rest -Si($R_5$)($R_6$)($R_7$) beispielsweise eine der Gruppen Trimethylsilyl, tris(tert.Butyl)silyl, Dimethyl-(2,3-dimethyl-2-butyl)-silyl, Diphenyl-tert.butylsilyl, bis(Isopropyl)methylsilyl, Triphenylsilyl usw. und insbesondere tert.Butyldimethylsilyl bildet. Y stellt eine Silylabgangsgruppe dar. Hierzu zählen beispielsweise Bromid, Chlorid, Cyanid, Azid, Acetamid, Trifluoroacetat, Trifluormethansulfonat. Diese Aufzählung stellt keine Limitierung dar, der Fachmann kennt weitere typische Silylabgangsgruppen.

5-O-Silylierungen werden in wasserfreiem Milieu, vorzugsweise in inerten Lösungsmitteln und besonders bevorzugt in aprotischen Lösungsmitteln durchgeführt. Die Reaktion läuft vorteilhaft im Temperaturbereich +80 °C, bevorzugt bei +10 ° bis +40 °C, ab. Vorzugsweise wird eine organische Base zugegeben. Es kommen als solche beispielsweise tertiäre Amine wie Triethylamin, Triethylendiamin, Triazol und bevorzugt Pyridin, Imidazol oder 1,8-Diazabicyclo[5.4.0]undec-7-en (DBU) in Frage.

Die Entfernung dieser Silylreste in der 5-Position geschieht auf an sich bekannte Weise durch selektive milde Hydrolyse mit z.B. Arylsulfonsäure in alkoholischer Lösung oder nach einer anderen dem Fachmann geläufigen Methode.

Die Herstellung der Oxime [$R_4$ = -C(=N-OH)-] im Umfang der Formel III erfolgt durch Reaktion eines Derivats der Formel III, in welchem $R_4$ durch -C(O)-ersetzt ist, mit Hydroxylamin oder einem seiner Salze, vorzugsweise einem seiner Mineralsäuresalze, insbesondere seinem Hydrochlorid. Die Reaktion wird zweckmässigerweise in einem geeigneten Lösungsmittel, z.B. einem Niederalkanol, wie Methanol, Ethanol, Propanol; einer etherartigen Verbindung, wie Tetrahydrofuran oder Dioxan; einer aliphatischen Carbonsäure, wie Essigsäure oder Propionsäure; Wasser oder in Gemischen dieser Lösungsmittel untereinander oder mit anderen üblichen reaktionsinerten Lösungsmittels durchführt. Die Reaktionstemperaturen können in weiten Bereichen variieren. Man arbeitet zweckmässigerweise etwa im Bereich von +10 ° bis +100 °C. Wird Hydroxylamin in Form eines seiner Salze, z.B. als Hydrochlorid eingesetzt, so ist es vorteilhaft, wenn man zum Abfangen der Säure (z.B. HCl) eine der für solche Zwecke üblichen Basen zusetzt und gegebenenfalls in Gegenwart eines Wasserbinders, z.B. eines Molekularsiebes, arbeitet. Als geeignete Basen kommen organische und anorganische Basen in Betracht, z.B. tertiäre Amine wie Trialkylamine (Trimethylamin, Triethylamin, Tripropylamin usw.), Pyridin und Pyridinbasen (4-Dimethylaminopyridin, 4-Pyrrolidylaminopyridin usw.), Oxide, Hydride und Hydroxide, Carbonate und Hydrogencarbonate von Alkali- und Erdalkalimetallen (CaO, BaO, NaOH, KOH, NaH, $Ca(OH)_2$, $KHCO_3$, $NaHCO_3$, $Ca(HCO_3)_2$, $K_2CO_3$, $Na_2CO_3$), sowie Alkaliacetate wie $CH_3COONa$ oder $CH_3COOK$. Darüberhinaus eignen sich auch Alkalialkoholate wie $C_2H_5ONa$, n-$C_3H_7ONa$ usw. Bevorzugt wird Triethylamin.

Die Derivate der Formel III, in welchen $R_4$ für -C(O)- steht, lassen sich durch milde Oxidation mit z.B. Braunstein ($MnO_2$), $CrO_3$/Pyridin oder durch Oppenauer-Oxydation aus den entsprechenden natürlichen Antibiotika S541 (S541A, S541C, S541D) und aus den Milbemycinen der Formel M herstellen.

Verbindungen der Formel III, in welchen $R_4$ für -C(O)-$CH_2$O-C(O)-$CH_2CH_2$COOH oder -C(O)-$CH_2CH_2$-COOH steht, lassen sich analog bekannter Methoden durch Veresterung aus den entsprechenden Milbemycinen und aus den natürlichen Antibiotika S541 herstellen.

Das erfindungsgemässe Verfahren wird im einzelnen wie folgt durchgeführt:

Zur Herstellung von Verbindungen der Formel I oder von Verbindungen der Formel II oder von Gemischen beider bringt man eine Verbindung der Formel III und einen Biokatalysator, welcher zur stereospezifischen Einführung einer Hydroxigruppe in die 13$\beta$-Position und/oder einer 14,15-Epoxidierung der Verbindungen der Formel III oder Für beide Reaktionen geeignet ist, in unmittelbaren Kontakt miteinander und hält diesen Kontakt über einen Zeitraum aufrecht, der für die Hydroxilierung bzw. Epoxidierung ausreicht.

Am zweckmässigsten wird das Verfahren in der Weise ausgeführt, dass man einen Mikroorganismus, der in der Lage ist die erfindungsgemässe Hydroxilierungs-/Epoxidierungsreaktion durchzuführen, in Gegenwart einer Verbindung der Formel III unter kontrollierten Bedingungen kultiviert und die gemeinsame Inkubation von besagtem Mikroorganismus und seinem Substrat solange aufrechterhält bis ein Grossteil der zugesetzten Verbindung der Formel III, vorzugsweise 80-99,9%, in Verbindungen der Formel I oder II oder Gemische dieser Verbindungen umgewandelt sind.

Die Isolierung der so erhaltenen Verbindungen der Formeln I oder II oder der beide Verbindungen enthaltenen Gemische lässt sich auf an sich bekannte Weise, z.B. mit Hilfe der Kieselgelchromatographie, durchführen.

Als Substrat für die erfindungsgemässe Hydroxilierungsreaktion dienen Verbindungen der Formel III. Diese Verbindungen sind bekannt (s. beispielsweise Formel M) oder lassen sich analog zu bekannten Verfahren aus bekannten Verbindungen herstellen. Sie eignen sich zur Bekämpfung von Schädlingen an Tieren und Pflanzen und stellen ausserdem wertvolle Ausgangs- oder Zwischenprodukte bei der Herstellung von Verbindungen der Formel I dar.

Die Herstellung von Verbindungen der Formel I lässt sich auch in der Weise durchführen, dass statt des Mikroorganismus zur Hyroxilierung geeignete, von diesem Mikroorganismus stammende aktive Bestandteile (gemäss Definition von Seite 3) für die Hydroxilierung von Verbindungen der Formel III verwendet werden. Auf diese Weise lassen sich auch die Epoxi-Verbindungen der Formel II sowie Gemische beider Verbindungen herstellen.

Bei der Verwendung von mikrobiellen Sporen anstelle der vegetativen Zellstrukturen, werden besagte Sporen von Mikroorganismen, die zur stereospezifischen 13$\beta$-Hydroxilierung oder 14,15-Epoxidierung von

Verbindungen der Formel III oder zu beiden Reaktionen geeignet sind, geerntet und anschliessend zusammen mit einer Verbingung der Formel III für einen Zeitraum, der für die entsprechende(n) Reaktion-(en) ausreicht, inkubiert. Die Inkubation von Sporen und Substrat erfolgt vorzugsweise in Abwesenheit von Kulturmedium um ein Auskeimen der Sporen zu verhindern.

Ein weiterer Aspekt der vorliegenden Erfindung betrifft die Verwendung von vegetativen Mikroorganismen-Zellen, zellfreien Extrakten, Sporen, Enzymen sowie Enzymgemischen besagter Mikroorganismen, die zu einer stereospezifischen Einführung einer 13$\beta$-Hydroxigruppe oder zur 14,15-Epoxidierung der Verbindungen der Formel III oder zu beiden Reaktionen geeignet sind, in immobilisierter Form.

Die Immobilisierung besagter Biokatalysatoren kann analog zu an sich bekannten Verfahren durchgeführt werden.

Im Rahmen der vorliegenden Erfindung sind in erster Linie Verfahren zu nennen, die auf einer adsorptiven, ionischen oder kovalenten Bindung besagter Biokatalysatoren an feste, in der Regel wasserunlösliche Trägermaterialien, auf einer Quervernetzung von Biokatalysatoren durch bi- oder mehrfunktionelle Reagentien, einer Matrixumhüllung, einer Membranabtrennung oder einer Kombination zweier oder mehrerer oder oben genannten Verfahren beruhen.

Die adsorptive Bindung an wasserunlösliche Trägerstoffe (Adsorbentien) erfolgt in erster Linie aufgrund von van der Waal-Wechselwirkungskräften. Als Adsorbentien kommen zahlreiche anorganische und organische Verbindungen, desgleichen auch synthetische Polymer in Betracht.

Methoden für eine derartige Immobilisierung von Mikroorganismen sind bei van Haecht et al, 1985 (Hefezellen/Glas), Black et al, 1984 (Hefezellen/Edelstahl, Polyester), Wiegel und Dykstra, 1984 (Clostridien/Cellulose, Hemicellulose) Förberg und Häggström, 1984 (Clostridien/Holzspäne) sowie bei Ehrhardt und Rehm, 1985 (Pseudomonaden/Aktivkohle) beschrieben. Entsprechende Anagaben für die Verwendung durch adsorptive Bindung immobilisierter Enzyme finden sich bei Krakowiak et al, 1984 (Glucoamylase/Aluminiumoxid), Cabral et al, 1984 (Glucoamylase/Titanaktiviertes Glas), Miyawaki und Wingard 1984 (Glucoseoxidase/Aktivkohle), Kato und Horikoshi, 1984 (Glucosetransferase/synthetisches Harz) u.a.

Ionische Bindungen basieren auf elektrostatischen Anziehungen zwischen entgegengesetzt geladenen Gruppen des Trägermaterials (wie beispielsweise handelsüblichen Ionenaustauschern z.B. auf der Basis von Polysacchariden oder von synthetischen Harzen) und des zu bindenden Biokatalysators.

Auf ionischer Bindung basierende Methoden zur Immobilisierung von Mikroorganismen sind bei DiLuccio und Kirwan, 1984 (Azotobacter spec./Cellex E (Cellulose)) sowie bei Giard et al., 1977 (Tierische Zellen/DEAE-Sephadex) beschrieben.

Eine entsprechende Immobilisierung von Enzymen kann gemäss den Angaben bei Angelino et al., 1985 (Aldehydoxidase/Octylamino-Sepharose 4B), Hofstee, 1973 (Lactatdehydrogenase/Octylamino-Sephadex), Kühn et al., 1980 (Glucoseoxidase/DEAE-Sephadex, DEAE-Cellulose) u.a. durchgeführt werden.

Eine weitere Methode zur Immobilisierung beruht auf der Ausnützung von kovalenten Bindungskräften, die im allgemeinen zu festen Verknüpfungen von Biokatalysatoren untereinander oder zwischen Biokatalysator und Trägermaterial führen, wobei als Trägermaterialien, poröse Materialien, wie Gläser, Kieselerde oder andere, unlösliche, anorganische Materialien eingesetzt werden können.

Im Rahmen des erfindungsgemässen Verfahrens kann man die Mikroorganismen z.B. analog zu Messing und Oppermann, 1979 (Enterobakterien/Borosilikatglas; Hefezellen/Zirkonerde), Romanovskaya et al., 1981 (Methanbakterien/Silochrome), Navarro und Durand, 1977 (Hefezellen/poröse Kieselerde) immobilisieren.

Die Immobilisierung von Enzymen kann nach der Methode von Weetall und Mason, 1973 (Papain/Poröses Glas) sowie Monsan et al., 1984 (Invertase/Poröse Kieselerde) durchgeführt werden.

Im erfindungsgemässen Verfahren kommen nicht nur die bereits genannten Trägermaterialien für eine Immobilisierung in Betracht sondern eine ganze Reihe von natürlichen oder synthetischen Polymeren, wie z.B. Cellulose, Dextran, Stärke, Agarose usw. oder Polymere z.B. auf der Basis von Acryl- und Methacrylsäurederivaten, die üblicherweise bei der Herstellung reaktiver Copolymerisate Verwendung findet. Als reaktive Gruppen über die eine Bindung mit dem Biokatalysator vermittelt wird sind reaktionsfähige Dinitrofluorphenyl- oder Isothiocyanatgruppen vor allem Oxiran- und Säureanhydridgruppen zu nennen. Eine weitere Möglichkeit besteht in der Chloridaktivierung von Carboxyl-Gruppen tragenden Harzen, die beispielsweise unter den Handelsnamen Amberlite® XE-64 und Amberlite® IRC-50 kommerziell erhältlich sind.

Die Immobilisierung von Mikroorganismen mit Hilfe von natürlichen oder synthetischen Trägermaterialien kann wie bei Chipley, 1974 (Bacillus subtilis/Agarose), Gainer et al., 1980 (Azotobacter species/Cellulose), Jack and Zajic, 1977 (Micrococcus luteus/Carboxymethylcellulose), Jirku et al., 1980 (Hefezellen/Hydroxyalkylmethacrylat) sowie bei Shimizu et al., 1975 (Bakterienzellen/Ethylen-Maleinanhydrid-Copolymer) beschrieben, durchgeführt werden. Die Immobilisierung von Enzymen kann analog zu Cannon et al, 1984 (Lactatoxidase/Cellulose), Clark and Bailey, 1984 (Chymotrypsin/ Sepharose), Ibrahim et

al., 1985 (Epoxidhydrolase/Dextran); Beddows et al., 1981 (α-Galactosidase/Nylon-Acrylat-Copolymer), Raghunath et al., 1984 (Urease/Methylacrylat-Acrylat) u.a. erfolgen.

Bei der Quervernetzung ("cross linking") werden die Biokatalysatoren durch bi- oder mehrfunktionelle Reagentien, wie Glutardialdehyd, Diisocyanate u.a., miteinander verbunden und bilden hochmolekulare, typischerweise unlösliche, meist gelartige Aggregate.

Derartige Immobilisierungen von Mikroorganismen kann man analog zu De Rosa et al., 1981 (Bakterienzellen/Co-Crosslinking mit Hühnereiweiss durch Glutardialdehyd) durchführen.

Verfahren zur Immobilisierung von Enzymen die im Rahmen der vorliegenden Erfindung verwendet werden können, sind bei Barbaric et al., 1984 (Invertase/Vernetzung mit Adipinsäuredihydrazid), Talsky und Gianitsopoulos, 1984 (Chymotrypsin/Peptidbindung zwischen den Enzymmolekülen ohne Vernetzungsagenz), Workman und Day, 1984 (Inulinase/Vernetzung der enzymhaltigen Zellen mit Glutardialdehyd), Khan und Siddiqi, 1985 (Pepsin/Vernetzung mit Glutardialdehyd), Bachmann et al., 1981 (Glucoseisomerase/Co-Crosslinking mit Gelatine durch Glutardialdehyd), Kaul et al., 1984 (β-Galactosidase/Co-Corsslinking mit Hühnereiweiss durch Glutardialdehyd) beschrieben.

Die Matrixumhüllung beinhaltet den Einschluss der Biokatalysatoren in natürliche oder synthetische Polymer, die meist gelartige Struktur aufweisen. Besonders geeignet als Matrixmaterialien für den Einschluss von Zellen, Organellen und Sporen sind natürliche Polymere wie Alginat, Carrageenan, Pectin, Agar, Agarose oder Gelatine, da diese Verbindungen nicht toxisch und in ihrer Handhabung zellschonend sind.

Weiterhin kommen in Frage synthetische Polymere, wie beispielsweise Polyacrylamide, photovernetzte Harze u.a. Die Gestaltung der Matrixumhüllungen ist innerhalb weiter Grenzen variabel und kann beispielsweise Kugel-, Zylinder-, Faser- oder Foliengestalt umfassen.

Die Immobilisierung von Mikroorganismen mit Hilfe von natürlichen oder synthetischen Matrixmaterialien kann wie bei Mazumder et al., 1985 (Bakterienzellen/photovernetzte Harze), Bettmann und Rehm, 1984 (Bakterienzellen/Polyacrylamidhydrazid), Umemura et al., 1984 (Bakterienzellen/Carrageenan), Karube et al., 1985 (Bakterienprotoplasten/Agar-Acetylcellulose), Cantarella et al., 1984 (Hefezellen/Hydroxyethylmethacrylat), Qureshi und Tamhane, 1985 (Hefezellen/Alginat), Deo und Gaucher, 1984 (Schimmelpilzmycel/Carrageenan), Eikmeier und Rehm, 1984 (Schimmelpilzmycel/Alginat), Bihari et al., 1984(Schimmelpilzkonidien/Polyacrylamid), Vogel und Brodelius, 1984 (Pflanzenzellen/Alginat, Agarose), Nakajima et al., 1985 (Pflanzenzellen/Agar, Alginat, Carrageenan) beschrieben, durchgeführt werden.

Die Immobilisierung von Enzymen kann analog zu Mori et al., 1972 (Aminoacylase/Polyacrylamid) erfolgen.

Dei der Membranabtrennung handelt es sich um die Scheffung definierter, abgegrenzter Räume, in denen die Reaktion(en) abläuft (ablaufen).

Man unterscheidet die folgenden grundsätzlichen Varianten der Membranabtrennung

a) Mikroenkapsulierung
b) Liposomentechnik
c) Anwendung von Biokatalysatoren in Membranreaktoren.

Die vorstehend beschriebenen Immobilisierungsmethoden können auch untereinander kombiniert verwendet werden, wie beispielsweise die Adsorption und die Quervernetzung. Die Enzyme werden dabei zunächst an einen Träger adsorbiert und anschliessend durch ein bifunktionelles Reagenz miteinander vernetzt.

Die Inkubation der im Rahmen der vorliegenden Erfindung verwendeten Biokatalysatoren mit Verbindungen der Formel III zur stereospezifischen Einführung einer Hydroxigruppe in der 13β-Position und/oder zur 14,15-Epoxidierung kann mit Hilfe von Verfahren durchgeführt werden, wie sie in der angewandten Mikrobiologie üblich sind.

Neben der Verwendung von Schüttelkulturen sind dabei in erster Linie verschiedene Fermentersysteme zu nennen, die in der mikrobiologischen Forschung und der industriellen Produktion seit langem etabliert sind.

Hauptaufgabe der Bioreaktoren ist die Schaffung von optimalen hydrodynamischen Verhältnissen zur Erniedrigung der scheinbaren Michaeliskonstanten und zur Erhöhung der Reaktionsgeschwindigkeit.

Dies wird im wesentlichen erreicht durch Aufrechterhaltung einer ausreichenden Relativbewegung zwischen Biokatalysator und umgebendem Medium, was den externen Massentransfer soweit steigert, dass seine Behinderung praktisch keine Rolle mehr spielt.

Für das vorliegende Verfahren geeignete Reaktortypen umfassen beispielsweise Rührreaktoren, Schlaufenreaktoren, Bettreaktoren, Wirbelschichtreaktoren, Membranreaktoren sowie zahlreiche Reaktor-Sonderformen, beispielsweise Siebrührer-Reaktoren, Romboidreaktoren, Röhrenreaktoren u.a. (W. Hartmeier, Immobilisierte Biokatalysatoren, 1986; W. Crueger und A. Crueger, Biotechnologie-Lehrbuch der angewandten

Mikrobiologie, 1984; P. Präve et al., Handbuch der Biotechnologie, 1984).

Bevorzugt im Rahmen der vorliegenden Erfindung ist die Verwendung von Rührreaktoren.

Rührreaktoren gehören zu den am meisten verwendeten Reaktortypen in der biotechnologischen Fermentationstechnik. Dieser Reaktortyp gewährleistet eine schnelle und gute Durchmischung von Substrat und Biokatalysator durch hohe Rührleistungen sowie eine hohe Sauerstoffübergangsleistung.

Die Vorteile der Rührreaktoren liegen in der einfachen und damit kostengünstigen Konstruktion sowie in seinen gut erforschten Eigenschaften begründet.

Grundsätzlich kann man bei Verwendung von Rührreaktoren zwei Betriebsarten in Betracht; zum einen ein "ansatzweise" betriebenes Verfahren, das sog. "batch"-Verfahren, zum anderen ein kontinuierliches Verfahren.

Beim "batch"-Verfahren werden die Biokatalysatoren nach Beendigung des Verfahrens durch Separation oder Filtration abgetrennt und entweder verworfen (vegetative Zellen) oder in einem zweiten Ansatz erneut verwendet (immobilisierte Biokatalysatoren).

Bei Anwendung des kontinuerlichen Verfahrens erfolgt ein ständiger kontinuierlicher Austausch von neuem Substrat gegen das Endprodukt der Reaktion. Die Biokatalysatoren müssen dabei durch geeignete Massnahmen (Siebe, Filter, Rückführungseinrichtungen) am verlassen des Reaktors gehindert werden.

Die Züchtung vegetativer Mikroorganismenzellen im Rahmen der vorliegenden Erfindung erfolgt nach bekannten, allgemein gebräuchlichen Methoden, wobei aus Gründen der Praktikabilität vorzugsweise flüssige Nährmedien verwendet werden.

Die Zusammensetzung der Nährmedien variiert je nach verwendetem Mikroorganismus. Allgemein werden komplexe Medien mit wenig definierten, leicht assimilierbaren Kohlenstoff-(C-) und Stickstoff-(N-) Quellen bevorzugt, wie sie üblicherweise z.B. auch für die Antibiotikaproduktion eingesetzt werden.

Zusätzlich werden Vitamine und essentielle Metallionen benötigt, die aber in der Regel in den verwendeten komplexen Nährmedien in ausreichender Konzentration als Bestandteile bzw. Verunreinigungen enthalten sind.

Gegebenenfalls können besagte Bestandteile wie z.B. essentielle Vitamine sowie $Na^+$, $K^+$, $Ca^{2+}$, $Mg^{2+}$, $NH_4^{\oplus}$, $PO_4^{3-}$, $SO_4^{2-}$, $Cl^-$, $CO_3^{2-}$-Ionen und die Spurenelemente Cobalt und Mangan, Zink u.a. in Form ihrer Salze zugesetzt werden.

Als besonders geeignete Stickstoffquellen sind neben Hefe-Extrakten, Hefe-Hydrolysaten, Hefe-Autolysaten sowie Hefe-Zellen vor allem Sojamehl, Maismehl, Hafermehl, Edamin (enzymatisch verdautes Lactalbumin), Pepton, Caseinhydrolysat, Maisablaugen sowie Fleischextrakte zu nennen.

Die bevorzugte Konzentration für die genannten N-Quellen liegt zwischen 0.1 und 6 g/l.

Als C-Quelle kommen in erster Linie Glucose, Lactose, Sucrose, Dextrose, Maltose, Stärke, Cerelose, Cellulose sowie Malzextrakt in Frage. Der bevorzugte Konzentrationsbereich liegt zwischen 1.0 und 25 g/l. Die Verwendung von D-Glucose oder löslicher Stärke sowie von Cerelose als Kohlenstoffquelle ist von Vorteil für das nachfolgend beschriebene Hydroxilierungs-/Epoxidierungsverfahren, besonders wenn als Mikroorganismen Vertreter der Gattung Streptomyces eingesetzt werden. So sind beispielsweise folgende Kulturmedien für Vertreter der Gattung Streptomyces ausgezeichnet geeignet:

Medium 1

1,0 g lösliche Stärke
0,2 g Pepton
0,2 g Hefeextrakt
ad 1 l mit dest. Wasser, pH 7 mit NaOH, autoklavieren.

Medium 2

5,0 g D-Glucose
1,0 g Pepton
0,1 g Hefeextrakt
ad 1 l mit dest. Wasser, pH 7 mit NaOH, autoklavieren.

Medium 3

22,0 g Cerelose
4,0 g Oxo Lab Lemco (Oxoid-Handbuch, 2. Auflage, 1972)
5,0 g Pepton C
0,5 g Hefeextrakt
3,0 g Casitone (Difco Manual, 9th ed., Detroit, Difco Laboratories, 1969)
1,5 g Natriumchlorid
ad 1 l mit destilliertem Wasser, pH 7 mit NaOH, autoklavieren.

Die oben genannten Medien sind ebenso in hervorragender Weise für die Kultivierung von Vertretern der Gattung Aspergillus sowie für die Durchführung der Hydroxylierungs- und/oder der Epoxidierungsreaktionen geeignet.

Sowohl die oben gemachten allgemeinen Angaben über die Medienzusammensetzung als auch die hier im einzelnen aufgeführten Medien dienen lediglich der Erläuterung der vorliegenden Erfindung und haben keinen limitierenden Charakter.

Neben der Medienzusammensetzung spielt auch die Vorgehensweise bei der Medienherstellung eine wichtige Rolle und sollte für das jeweilige Produktionsverfahren entsprechend optimiert werden, wie beispielsweise die Reihenfolge der Lösung oder Suspendierung, die Sterilisation der gesamten Nährlösung oder aber einzelner Bestandteile, die Verhinderung von Verunreinigungen u.a.

Es ist weiterhin zu beachten, dass es infolge der Sterilisation zu Veränderungen des pH-Wertes, des Nährmediums sowie zu Ausfällungen kommen kann.

Die übrigen Kultivierungsmethoden entsprechen ebenfalls den üblicherweise für die Kultivierung von Mikroorganismen angewendeten Verfahren.

Im kleinen Masstab werden die Fermentationen im Rahmen der vorliegenden Erfindung gewöhnlich in Form von Schüttelkulturen durchgeführt. Dabei werden zweckmässigerweise Glaskolben mit einem Volumen von 0.5 bis 5 Litern verwendet, die zwischen 0.1 und 2 Litern Nährmedium enthalten. Nach dem Autoklavieren und Einstellen des pH-Wertes auf Werte von pH 4 bis pH 8, insbesondere auf Werte von pH 7.0 bis pH 7.4, (Bakterien) bzw. auf Werte von pH 6 bis pH 7.5 (Pilze) werden die Kolben mit den entsprechenden Mikroorganismenkulturen unter sterilen Bedingungen beimpft. Als Impfmaterial dienen in der Regel Vorkulturen, die gemäss den unten gemachten Angaben aus Impfgutkonserven hergestellt werden.

Die Kulturen werden zweckmässigerweise unter aeroben Bedungungen und bei einer Temperatur von 25 bis 37 °C, insbesondere 28 °C, unter ständigem Schütteln bei 250 Upm auf der Rundschüttelmaschine angezogen. Nach 24 Stunden erfolgt die Zugabe des Substrats (Verbindungen der Formel III), wobei Substanz auf beliebige Weise miteinander in Kontakt gebracht werden können. Aus Gründen der Praktischen Handhabung erweist es sich als günstig, das Substrat, also eine Verbindung der Formel III, dem in Nährlösung befindlichen Mikroorganismus beizufügen. Die zu hydroxilierende epoxidierende Substanz kann beispielsweise in Pulverform oder als Lösung in einem geeigneten Lösungsmittel wie beispielsweise Dimethylformamid, Aceton oder Dimethylsulfoxyd oder einem alkoholischen Lösungsmittel wie beispielsweise Methanol, Ethanol oder tert.-Butanol eingesetzt werden (0,5 bis 15 Vol.%, vorzugsweise 2 Vol.%).

Der Reaktionsverlauf wird mittels chromatographischer Methoden, die in der mikrobiologischen Forschung allgemein Anwendung finden, ständig kontrolliert.

Unter den oben genannten Bedingungen wird im allgemeinen nach 2- bis 6-tägiger Kultivierung eine optimale Hydroxilierungs- bzw. Epoxidierungsaktivität mit Streptomyces erzielt. Es hat sich gezeigt, dass optimale Züchtungsbedingungen für die Mikroorganismen auch die optimale Hydroxilierungs- und Epoxidierungsbedingungen darstellen.

Ein weiterer Bestandteil der vorliegenden Erfingung betrifft die Kultivierung von Mikroorganismen, die zur stereospezifischen Einführung einer Hydroxigruppe in 13$\beta$-Position oder zu einer 14,15-Epoxidierung von Verbindungen der Formel III oder zu beiden Reaktionen in der Lage sind sowie deren Inkubation mit besagten Verbindungen in Bioreaktoren, insbesondere in Bioreaktoren vom Rührreakter-Typ.

Um eine optimale Produktbildungsrate im eigentlichen Produktionsfermenter zu gewährleisten empfiehlt es sich, dass die Mikroorganismen zunächst in Vorkulturen vermehrt werden. Die Anzahl der Fermenter-Vorkulturen hängt von der jeweils optimalen Impfgut-Konzentration ab. Zweckmässigerweise werden für eine Fermenterstufe, abhängig von den eingesetzten Mikroorganismen, folgende Impfgutkonzentrationen hergestellt:
Bakterien 0,1-3 %, Pilze 5-10 %.
Actinomycetales 5-10 %, Sporensuspensionen 1-5 x $10^5$ l/Kulturlösung
Die Beimpfung von Kleinfermentern (bis 20 l) erfolgt gewöhnlich unter Verwendung von Schüttelkolben-Vorkulturen. Dabei wird der gesamte Kolbeninhalt zur Beimpfung des Fermenters verwendet.

Als Ausgangsmaterial für die Herstellung von Vorkulturen dienen in der Regel Impfgutkonserven, die beispielsweise in Form von Lyophilisaten, von gefrorenen oder kühl gelagerten Konserven vorliegen können. Im Rahmen der vorliegenden Erfindung werden vorzugsweise Lyophilisate als Impfgutkonserven verwendet.

Die Impfgutvermehrung wird vorzugsweise in flüssigen Medien in Glaskolben auf der Schüttelmaschine bzw. bei Verwendung von Sporen auf festen Nährböden durchgeführt. Dabei müssen je nach verwendetem Mikroorganismus oder Verfahren die Bedingungen in Bezug auf Nährböden und Kultivierungsparameter, wie Temperatur, pH, Sauerstoffeintrag u.a., optimiert werden. Die Anwachszeiten für die Impfgutkonserven schwanken je nach verwendetem Ausgangsmaterial zwischen wenigen Stunden und mehreren Tagen.

| | |
|---|---|
| Lyophilisate | 3-10 Tage |
| gefrorene Konserven Bakterien | 4-18 Stunden |
| Actinomycetales | 1-5 Tage |
| Pilze | 1-7 Tage |
| kühl gelagerte Kulturen Bakterien | 4-24 Stunden |
| Actinomycetales | 1-3 Tage |
| Pilze | 1-5 Tage |

Bei Verwendung von Sporen als Impfmaterial werden die Sporen zunächst aus Impfgutkonserven auf festen Nährböden unter standardisierten Bedingungen (sterile Belüftung, Klimakammer) vermehrt. Bei Verwendung von lockerem Nährboden auf Torf-, Kleie-, Reis- oder Gersten-Basis werden die Kulturen zur Erzielung hoher Sporendichten, täglich durchgeschüttelt. Eine weitere Möglichkeit besteht in der Kultivierung der Impfgutkonserven auf durch Agar oder andere gebräuchliche Gelierungsmittel verfestigten Nährmedien, wobei bevorzugt solche Nährmedien verwendet werden, die eine Induktion der Sporenbildung auslösen.

Die Sporulationszeit beträgt je nach verwendetem Mikroorganismus und in Abhängigkeit von dem eingesetzten Nährmedium zwischen 7 und 30 Tagen.

Zur Beimpfung der Vorkultur- oder Produktionsfermenter werden die Sporen entweder mit oberflächenaktiven Mitteln, beispielsweise einer Tween 80-Lösung, aufgeschwemmt und mitsamt ihrem Nähremedium in den Fermenter transferiert, oder bei Verwendung fester Nährmedien, ebenfalls mit Hilfe besagter oberflächenaktiver Mittel von den festen Nährböden abgespült. Die so gewonnene sporenhaltige Lösung wird dann für die Beimpfung der Fermenter eingesetzt. Sowohl die Gewinnung der Sporen als auch die Beimpfung der Fermenter wird vorzugsweise unter sterilen Bedingungen durchgeführt.

Für die Herstellung von Verbindungen der Formel I und II im Rahmen der vorliegenden Erfindung können je nach benötigter Produktmenge Bioreaktoren unterschiedlicher Dimension eingesetzt werden, die grössenordnungsmässig Volumina zwischen 0,001 $m^3$ bis 450 $m^3$ umfassen.

Bei Verwendung gerührter Bioreaktoren sind die folgenden Fermentationsparameter für einen optimalen Reaktionsablauf als kritisch zu betrachten:

1. Temperatur: Die mikrobielle Hydroxilierungs- und/oder Epoxidierungsreaktion im Rahmen des erfindungsgemässen Verfahrens wird vorzugsweise im mesophilen Temperaturbereich (Temperaturbereich 20-45 °C) durchgeführt. Der für das Wachstum und die Produktbildung optimale Temperaturbereich liegt bei Werten zwischen 20 bis 32 °C, insbesondere bei Werten zwischen 24-30 °C.

2. Belüftung: Die Belüftungsrate liegt bei 0,1-1,0 vvm (Volumen Luft pro Volumen Flüssigkeit pro Minute) vorzugsweise bei 0,3 bis 0,6 vvm. Die Belüftungsrate muss gegebenenfalls im Verlaufe der Fermentation dem erarbeiteten $O_2$-Bedarf angepasst werden.

3. Druck: Die Rührreaktoren werden in der Regel unter leichtem Ueberdruck von 0,2 bis 0,5 bar gefahren um die Kontaminationsgefahr zu verringern.

4. pH-Wert: Je nach verwendetem Mikroorganismus kann der pH-Wert innerhalb bestimmter Grenzen schwanken. Beim Einsatz von Mikroorganismen aus der Gruppe der Actinomycetin liegt der Ausgangs-pH-Wert bei Werten zwischen pH 6 und pH 8 vorzugsweise bei Werten zwischen pH 7 und pH 7.4.

Bei Verwendung von Pilzen, insbesondere bei Pilzen aus der Gruppe der Moniliales liegt der Ausgangs-pH der Kulturlösung vorzugsweise zwischen Werten von pH 4 und pH 8 insbesondere bei Werten zwischen pH 6 und pH 7.5.

5. Rührung: Die Rührgeschwindigkeit ist abhängig vom verwendeten Rührertyp und der Fermentergrösse. Im Rahmen der vorliegenden Erfindung werden Rührer vom Scheibentyp bevorzugt die bei einer Grösse des Rührreaktors von 0.002 $m^3$ mit Geschwindigkeiten zwischen 250-450 Upm, insbesondere

zwischen 350-400 Upm betrieben werden.

Die Fermentationsdauer im Rahmen der vorliegenden Erfindung schwankt je nach verwendetem Mikroorganismus zwischen 5 bis 10 Tagen. Die Fermentation wird abgebrochen, wenn ein Grossteil (80-99 %) des zu Beginn zugegebenen Substrats (Verbindungen der Formel III) in Verbindungen der Formel I oder II oder ein Gemisch beider Verbindungen umgewandelt ist.

Um den optimalen Zeitpunkt für eine Beendigung der Hydroxilierungs- und/oder der Epoxidierungsreaktion zu ermitteln, wird während der gesamten Fermentation der Reaktionsverlauf mit Hilfe üblicherweise verwendeter Analyseverfahren, insbesondere chromatographischer Verfahren, wie beispielsweise HPLC oder dünnschichtchromatographische Verfahren, kontrolliert.

Die Aufarbeitung der Fermentationsbrühe zur Gewinnung der Hydroxilierungs- oder der Epoxidierungsprodukte oder eines Gemisches beider Produkte kann mit den üblicherweise in der Fermentationstechnik benützten Verfahren durchgeführt werden (W. Crueger und A. Crueger, 1984; P. Präve, 1984).

Zunächst werden die partikulären Bestandteile aus der Fermentationsbrühe mit Hilfe von Filtern, Zentrifugen oder Separatoren abgetrennt.

Beim Einsatz vegetativer Zellen muss der innerhalb der Zelle vorliegende Anteil der Reaktionsprodukte gewonnen werden. Dafür stehen verschiedene Zellaufschluss-Methoden zur Verfügung, die auf mechanischen, thermischen, chemischen oder enzymatischen Verfahren beruhen.

Als mechanische Methoden, die für eine Anwendung in dem erfindungsgemässen Verfahren in Frage kommen, seien hier beispielhaft das Vermahlen in Rührwerkskugelmühlen oder Kolloid-Mühlen, die Anwendung von Druck und Entspannung im Homogenisator sowie der Zellaufschluss durch Einwirkung von Ultraschall genannt. Zu den nicht-mechanischen Verfahren zählen der Zellaufschluss durch Trocknung, Lyse der Zellen durch osmotischen Schock, chemische Autolyse sowie die enzymatische Lyse der Zellen.

Nach der Abtrennung der partikulären Bestandteile erfolgt die Anreicherung der Reaktionsprodukte durch Extraktion der Kulturlösung und der abgetrennten zellulären Bestandteile. Für die Extraktion stehen ebenfalls zahlreiche Hilfsmittel zur Verfügung, die in der Fermentationstechnik üblicherweise Anwendung finden, wie z.B. Misch-Abscheider, Gegenstromkolonnen, Extraktionszentrifugen, u.a.m.

Einer weitere Möglichkeit der Anreicherung der Reaktionsprodukte bieten beispielsweise die Membranfiltration, Ultrafiltration, Gefrierkonzentrierung, Ionenaustauschverfahren, u.a.m.

Die weitere Aufarbeitung des nach der Extraktion gewonnenen Rohprodukte kann mit Hilfe von Verfahren durchgeführt werden, die in der mikrobiologischen bzw. chemischen Forschung und der industriellen Anwendung fest etabliert sind.

Dazu erhören z.B. chromatographische Verfahren, wie die Adsorptionschromatographie. Ionenaustauschchromatographie, die Molekularsiebchromatographie, die Affinitätschromatographie, hydrophobe Chromatographie, Verteilungschromatographie, kovalente Chromatographie u.a.m., daneben aber auch verschiedene Kristallisationsverfahren.

Wie eingangs bereits angedeutet, liefert das erfindungsgemässe Verfahren sowohl Verbindungen der Formel I als auch Verbindungen der Formel II. Im allgemeinen erhält man Gemische hydroxilierter und epoxidierter Verbindungen, wobei das Verhältnis von hydroxilierter Verbindung zu epoxidierter Verbindung durch die Wahl des Mikroorganismus, des Ausgangsproduktes und die Reaktionsbedingungen (Nährlösung, organisches Lösungsmittel, Zugaberate des Substrats, Belüftung) beeinflusst sein kann. Die in einem Gemisch vorliegenden Verbindungen unterscheiden sich nur im Bereich Atome C-13, C-14 und C-15:

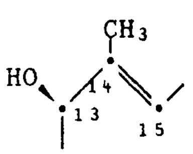

I
hydroxiliert

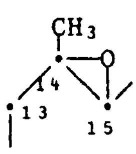

II
epoxidiert

und weisen ansonsten keine weiteren Unterschiede in der Molekülstruktur auf. Die hydroxilierten und epoxidierten Verbindungen lassen sich, wie bereits gesagt, leicht isolieren und voneinander abtrennen oder man kann (II) in (I) überführen.

Die Verbindungen der Formeln I und II, einschliesslich der neuen Oxime im Umfang der Formeln I und II, eignen sich ausgezeichnet zur Bekämpfung von Schädlingen an Tieren und Pflanzen, darunter tierparasitären Ekto-Parasiten. Zu letzteren zählen unter der Ordnung Acarina insbesondere Schädlinge der Familien

Ixodidae, Dermanyssidae, Sarcoptidae, Psoroptidae; die Ordnungen Mallophaga; Siphonaptera, Anoplura - (z.B. Familie der Haematopinidae); unter der Ordnung Diptera insbesondere Schädlinge der Familien Muscidae, Calliphoridae, Oestridae, Tabanidae, Hippoboscidae, Gastrophilidae.

Die Verbindung der Formeln I und II sind auch einsetzbar gegen Hygiene-Schädlinge, insbesondere der Ordnungen Diptera mit den Familien Sarcophagidae, Anophilidae, Culicidae; der Ordnung Orthoptera, der Ordnung Dictyoptera (z.B. Familie Blattidae) und der Ordnung Hymenoptera (z.B. Familie Formicidae).

Die Verbindungen der Formeln I und II besitzen auch nachhaltige Wirksamkeit bei pflanzenparasitären Milben und Insekten. Bei Spinnmilben der Ordnung Acarina sind sie wirksam gegen Eier, Nymphen und Adulte von Tetranychidae (Tetranychus spp. und Panonychus spp.).

Hohe Aktivität besitzen sie bei den saugenden Insekten der Ordnung Homoptera, insbesondere gegen Schädlinge der Familien Aphididae, Delphacidae, Cicadellidae, Psyllidae, Coccidae, Diaspididae und Eriophydidae (z.B. die Rostmilbe auf Citrusfrüchten): Der Ordnungen Hemiptera; Heteroptera und Thysanoptera; sowie bei den pflanzenfressenden Insekten der Ordnungen Lepidoptera, Coleoptera; Diptera und Orthoptera.

Sie sind ebenfalls als Bodeninsektizid gegen Schädlinge im Erdboden geeignet.

Die Verbindungen der Formeln I und II sind daher gegen alle Entwicklungsstadien saugender und fressender Insekten an Kulturen wie Getreide, Baumwolle, Reis, Mais, Soja, Kartoffeln, Gemüse, Früchten, Tabak, Hopfen, Citrus, Avocados und anderen wirksam.

Die Verbindungen der Formeln I und II sind auch wirksam gegen Pflanzen-Nematoden der Gattungen Meloidogyne, Heterodera, Pratylenchus, Ditylenchus, Radopholus, Rizoglyphus und andere.

Besonders aber sind die Verbindungen gegen Helminthen wirksam, unter denen die endoparasitären Nematoden die Ursache schwerer Erkrankungen an Säugetieren und Geflügel sein können, z.B. an Schafen, Schweinen, Ziegen, Rindern, Pferden, Eseln, Hunden, Katzen, Meerschweinchen, Ziervögeln. Typische Nematoden dieser Indikation sind: Haemonchus, Trichostrongylus, Ostertagia, Nematodirus, Cooperia, Ascaris, Bunostonum, Oesophagostonum, Charbertia, Trichuris, Strongylus, Trichonema, Dictyocaulus, Capillaria, Heterakis, Toxocara, Ascaridia, Oxyuris, Ancylostoma, Uncinaria, Toxascaris une Parascaris. Der besondere Vorteil der Verbindungen der Formeln I und II ist ihre Wirksamkeit gegen solche Parasiten, die gegen Wirkstoffe auf Benzimidazol-Basis resistent sind.

Gewisse Spezies der Gattungen Nematodirus, Cooperia und Oesophagostomum greifen den Intestinaltrakt des Wirtstiers an, während andere der Gattungen Haemonchus und Ostertagia im Magen und solche der Gattung Dictyocaulus im Lungengewebe parasitieren. Parasiten der Familien Filariidae und Setariidae finden sich im internen Zellgewebe und den Organen, z.B. dem Herzen, den Blutgefässen, den Lymphgefässen und dem subcutanen Gewebe. Hier ist vor allem der Herzwurm des Hundes, Dirofilaria immitis, zu nennen. Die Verbindungen der Formeln I und II sind gegen diese Parasiten hoch wirksam.

Sie sind ferner zur Bekämpfung von humanpathogenen Parasiten geeignet, unter denen als typische, im Verdauungstrakt vorkommende Vertreter solche der Gattungen Ancylostoma, Necator, Ascaris, Strongyloides, Trichinella, Capillaria, Trichuris und Enterobius zu nennen sind. Wirksam sind die Verbindungen vorliegender Erfindung auch gegen Parasiten der Gattungen Wuchereria, Brugia, Onchocerca und Loa aus der Familie der Filariidae, die im Blut, im Gewebe und verschiedenen Organen vorkommen, ferner gegen Dracunculus und Parasiten der Gattungen Strongyloides und Trichinella, die speziell den Gastro-Intestinalkanal infizieren.

Die Verbindungen der Formel I oder der Formel II oder Mischungen beider werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Verbindungen der Formeln I und II werden bei Warmblütern in Aufwandmengen von 0,01 bis 10 mg/kg Körpergewicht angewendet, über geschlossenen Kultur-Anbauflächen, in Pferchen, Stallungen oder sonstigen Räumen in Mengen von 10 g bis 1000 g pro Hektar.

Die Formulierungen, d.h. die den Wirkstoff der Formeln I oder II oder Mischungen beider enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie

Cyclohexanon, stark polare Lösungsmittel wie N-Methyl-2-pyrrolidon,Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialen anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffe nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen, Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Die in der Formulierungstechnik gebräuchlichen Tenside sind u.a. in folgender Publikation beschrieben:

"1986 International McCutcheon's Emulsifiers and Detergents"
The Manufacturing Confectioner Publishing Co.,
Glen Rock, New Jersey, USA.

Die pestiziden Zubereitungen enthalten in der Regel 0,01 bis 95 %, insbesondere 0,1 bis 80 %, Wirkstoff der Formel I oder der Formel II oder eines Gemisches beider, 5 bis 99,9 % eines festen oder flüssigen Zusatzstoffes und 0 bis 25 %, insbesondere 0,1 bis 25 %, eines Tensides. Liegt ein Gemisch von Verbindung I und Verbindung II vor, so beträgt das Verhältnis von I:II im allgemeinen zwischen 99,9:0,1 und 0,1:99,9.

Während als Handelsware eher konzentrierte Mittel bevorzugt werden, verwendet der Endverbraucher in der Regel verdünnte Mittel mit 1-10'000 ppm Wirkstoffgehalt.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

## Herstellungsbeispiele

### H1. Herstellung einer Vorkultur von Streptomyces violascens

Eine lyophiliserte Probe von S. violascens (ATCC 31560) wird in einen 50 ml Schüttelkolben zu 20 ml Medium 1 gegeben und 72 Stunden bei 28°C und 250 Upm inkubiert. Anschliessend werden 2 ml dieser ersten Vorkultur in einen 500 ml Schüttelkolben mit 200 ml Nährlösung Medium 1 bei 28°C und 250 Upm 48 Stunden inkubiert.

Auf entsprechende Weise können auch Vorkulturen von S. violascens (ATCC 31560), S. diastatochromogenes (ATCC 31561) und A. niger (ATCC 20567) unter Verwendung der Medien 1, 2 und 3 hergestellt werden.

H2. Die Kultivierungsbedingungen für die Hydroxilierung von Milbemycin $A_4$ im Schüttelkolben

Eine gemäss H1 hergestellte, 2 Tage alte Vorkultur von S. violascens in 450 ml Medium 2 wird mit 0,2 g Milbemycin $A_4$ versetzt und bei 28 °C und 250 Upm geschüttelt. Der Reaktionsablauf wird mittels HPLC (high pressure liquid chromatography) kontrolliert (Säule 25 cm/0,5 cm, Laufmittel Hexan/Dimethoxyäthan 3:1, 1 ml/min. UV-Detektor 220 nm). Nach 4 Tagen Schütteln und 37 % Umsatz entstehen 70 % 13β-Hydroxymilbemycin $A_4$ sowie 30 % 14,15-Epoxymilbemycin $A_4$.

Zur Aufarbeitung wird das Reaktionsgemisch mit 200 ml Diethyläther ($Et_2O$) versetzt und durch Hyflo Supercel® (Super Cel; Kieselgur gereinigt und geglüht; vorherrschende Partikelgrösse 2-25 μ: Fluka Katalog Nr. 56678) filtriert. Der Filterrückstand wird sorgfältig mit 100 ml $Et_2O$ gewaschen. Die Wasserphase wird 2 mal mit je 200 ml $Et_2O$ extrahiert. Die vereinigten Aetherphasen werden über $MgSO_4$ getrocknet und im Vakuum eingeengt. Das resultierende Rohprodukt (0,21 g) wird an $SiO_2$ (Kieselgel 60, 0,040-0,063 mm) mit Hexan/Essigester 1:1 chromatographiert (W. Clark-Still et al., J. Organ. Chem. 43, pp. 2923, 1978) und die einzelnen Produkte werden mittels 300 MHz $^1$H-NMR identifiziert.

In einem weiteren Versuch liefert dieses Verfahren mit Milbemycin $A_4$ bei einem Umsatz von 43 % Anteile von 78 % 13β-Hydroxymilbemycin $A_4$ und von 22 % 14,15-Epoxymilbemycin $A_4$ (HPLC-Analyse).

H3. Hydroxylierung von Milbemycin $A_4$ in Medium 3 mit 2,5 % DMSO

Durch Variation bestimmter Parameter [z.B. der DMSO(Dimethylsulfoxid)-Konzentration] lässt sich das Gleichgewicht zwischen den Reaktionsprodukten (13β-Hydroxy- und 14,15-Epoxymilbemycin) in Richtung der Hydroxiverbindung verschieben.

Eine 1 Tag alte Kultur von S. violascens in 200 ml Medium 3 wird mit 0,25 g Milbemycin $A_4$ und 5 ml DMSO versetzt und bei 28 °C und 250 Upm geschüttelt. Nach 3 Tagen werden weitere 50 ml Medium 3 zugegeben. Der Reaktionsablauf wird mittels HPLC (Säule Kontron LiSi 60, 25 cm/0,5 cm, Laufmittel Hexan/Dimethoxyethan 3:1, 1 ml/min, UV-Detektor 220 nm) kontrolliert. Nach 7 Tagen Schütteln und 91 % Umsatz entstehen 92 % 13β-Hydroxymilbemycin $A_4$ sowie 8 % 14,15-Epoxymilbemycin $A_4$.

H4. Hydroxylierung von Milbemycin $A_3$ und Milbemycin D

Werden Milbemycin $A_3$ oder Milbemycin D auf analoge Weise umgesetzt, so erhält man beispielsweise bei einem Umsatz von 10 %, Anteile von 40 % 13β-Hydroxymilbemycin $A_3$ und von 60 % 14,15-Epoxymilbemycin $A_3$ oder bei einem Umsatz von 13 %, Anteile von 29 % 13β-Hydroxymilbemycin D und von 71 % 14,15-Epoxymilbemycin D (HPLC-Analyse).

H5. Hydroxylierung von Milbemycin $A_4$-5-oxim

Auf analoge Weise wie vorstehen beschrieben lassen sich auch 13-Deoxy-22,23-dihydro-C-076-Bla-aglykon und Verbindungen der Formeln I, in denen A für

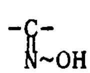

steht, in die entsprechenden 13β-Hydroxy-Verbindungen überführen.

Eine 1 Tag alte Kultur von S. violascens in 200 ml Medium 3 wird mit 0,25 g Milbemycin $A_4$-5-oxim und 5 ml Dimethylsulfoxid (DMSO) versetzt und bei 28 °C und 250 Upm geschüttelt. Der Reaktionsablauf wird mittels Dünnschichtchromatographie (Fertigplatten Kieselgel 60F Merck, Laufmittel Hexan/Essigester 1:1) kontrolliert. Nach 9 Tagen Schütteln ist kein Umsatz mehr festzustellen. Zure Aufarbeiting wird das Reaktionsgemisch mit 100 ml Dichloromethan ($CH_2Cl_2$) versetzt und durch Hyflo Supercel® filtriert. Der Filterrückstand wird mit 50 ml $CH_2Cl_2$ gewaschen. Die Wasserphase wird 3 mal mit je 100 ml $CH_2Cl_2$ extrahiert. Die organischen Phasen werden vereinlgt, über $MgSO_4$ getrocknet und i.V. eingeengt. Das resultierende Rohprodukt wird über $SiO_2$ (Kieselgel 60 Merck, 0,040-0,063 mm) mit Hexan/Essigester 1:1 chromatographiert. Das 13β-Hydroxymilbemycin $A_4$-5-oxim wurde mit 26 % Ausbeute isoliert.

H6. Kultivierungsbedingungen für die Hydroxilierung von Milbemycin $A_4$ im Rührreaktor

Für die Durchführung der Hydroxylierungsreaktion von Verbindungen der Formel III werden Bioreaktoren von Rührreaktor-Typ der Firma MBR (Schweiz), Modell Mini verwendet. Diese Reaktoren haben ein Fassungsvermögen von 2 Litern und sind mit Scheiben-Rührern ausgestattet. Die Belüftung erfolgt mittels steril-gefilterter Pressluft.

Vor dem Beginn der Fermentation werden der Reaktor und das Kultur-Medium (medium 3) getrennt autoklaviert. Der pH-Wert wird anschliessend unter sterilen Bedingungen durch Zugabe einer sterilen 2N NaOH-Lösung auf einen Wert von pH 7,0-7,4 eingestellt. Nach dem Abkühlen des Fermentationsmediums wird die Fermentation durch Zugabe von 200 ml einer 3 Tage alten Vorkultur von S. violascens in Medium 3 sowie durch Zugabe von 1,5 g Milbemycin $A_4$, gelöst in 10 ml DMSO, gestartet. Auch das Beimpfen des Produktionsfermenters erfolgt unter sterilen Bedingungen.

Die Fermentation wird bei einer Temperatur von 28 °C und einer Belüftungsrate von 0,44 vvm durchgeführt. Die Rührergeschwindigkeit beträgt 400 Upm. Zu dem Fermentationsmedium werden während der gesamten Inkubationsdauer täglich 100 ml frisches Kulturmedium 3 unter sterilen Bedingungen zugegeben.

Der Reaktionsverlauf wird während der gesamten Fermentation mit Hilfe chromatographischer Analysen (HPLC) verfolgt. Nach einer Gesamt-Inkubationszeit von 9 Tagen sind 86 % des zu Beginn der Fermentation zugegebenen Milbemycin $A_4$ umgesetzt. 61 % des umgesetzten Milbemycin $A_4$ konnten mittels HPLC als 13$\beta$-Hydroxy-Milbemycin $A_4$ identifiziert werden, 8 % als 14,15 Epoxymilbemycin $A_4$.

Nach Beendigung der Fermentation wird das Reaktionsgemisch mit 400 ml Methylenchlorid ($CH_2Cl_2$) versetzt und durch Hyflo Supercel® filtriert. Das so gewonnene Filtrat wird anschliessend kontinuierlich mit Methylenchlorid extrahiert. Nach 48 Stunden wird die organische Phase abgetrennt, über $MgSO_4$ getrocknet und in Vakuum eingeengt.

Das resultierende Rohprodukt wird dann entsprechend dem in Beispiel H2. beschriebenen Verfahren mit Hilfe chromatographischer Methoden gereinigt.

Tabelle 1: Typische Vertreter von Verbindungen der Formel I

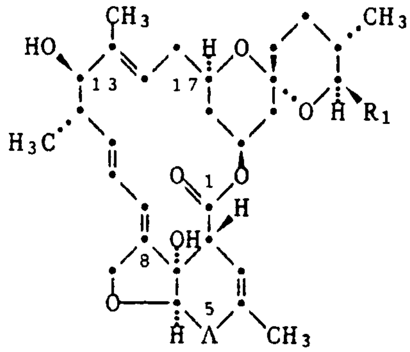

(I)

| Verb. Nr. | $R_1$ | Λ |
|---|---|---|
| 1.1 | $C_2H_5$ | 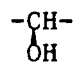 |
| 1.2 | $CH_3$ | 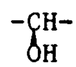 |
| 1.3 | iso$C_3H_7$ | 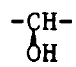 |
| 1.4 | sek.$C_4H_9$ | 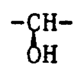 |
| 1.5 | $CH_3$ | 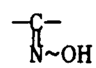 |
| 1.6 | iso$C_3H_7$ | 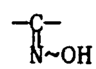 |
| 1.7 | $C_2H_5$ | 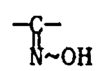 |
| 1.8 | sek.$C_4H_9$ | 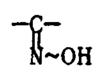 |

Tabelle 2: Typische Vertreter von Verbindungen der Formel II

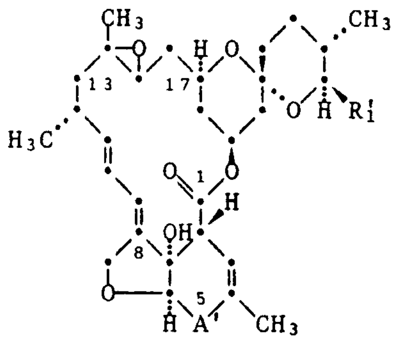

(II)

| Verb. Nr. | $R_1'$ | A' |
|---|---|---|
| 2.1 | $C_2H_5$ | 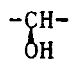 |
| 2.2 | $CH_3$ | 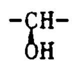 |
| 2.3 | iso$C_3H_7$ | 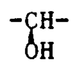 |
| 2.4 | sek.$C_4H_9$ | 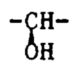 |
| 2.5 | $CH_3$ | 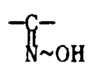 |
| 2.6 | iso$C_3H_7$ | 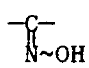 |
| 2.7 | $C_2H_5$ | 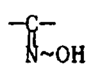 |
| 2.8 | sek.$C_4H_9$ | 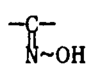 |

Formulierungsbeispiele

(% = Gewichtsprozent)

F1. Spritzpulver

| | a) | b) | c) |
|---|---|---|---|
| Wirkstoff aus den Tabellen 1 und 2 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykol-ether (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

F2. Emulsions-Konzentrat

| | |
|---|---|
| Wirkstoff aus den Tabellen 1 und 2 | 10 % |
| Octylphenolpolyethylenglykolether (4-5 Mol AeO) | 3 % |
| Ca-Dodecylbenzolsulfonat | 3 % |
| Ricinusölpolyglykolether (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

F3. Stäubemittel

| | a) | b) |
|---|---|---|
| Wirkstoff aus den Tabellen 1 und 2 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

F4. Extruder Granulat

| | |
|---|---|
| Wirkstoff aus den Tabellen 1 und 2 | 10 % |
| Na-Ligninsulfonst | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

F5. Tabletten bzw. Boli

| | | |
|---|---|---|
| I | Ein Wirkstoff aus den Tabellen 1 und 2 | 33,0 % |
| | Methylcellulose | 0,80 % |
| | Kieselsäure hochdispers | 0,80 % |
| | Maisstärke | 8,40 % |

Methylcellulose in Wasser einrühren und quellen lassen; Kieselsäure in die Quellung einrühren und homogen suspendieren. Wirkstoff und Maisstärke mischen. In diese Mischung die wässrige Suspension einarbeiten und zu einem Teig kneten. Diese Masse durch ein Sieb (Maschenweite 12 M) granulieren und dann trocknen.

| | | |
|---|---|---|
| II | Milchzucker krist. | 22,50 % |
| | Maisstärke | 17,00 % |
| | mikrokrist. Cellulose | 16,50 % |
| | Magnesiumstearat | 1,00 % |

Alle 4 Hilfsstoffe gut mischen.

Phasen I und II mischen und zu Tabletten oder Boli verpressen.

In den Fällen, in denen die Wirkstoff bzw. entsprechende Mittel zur Bekämpfung von endoparasitären Nematoden, Cestoden und Trematoden bei Haus- und Nutztieren, wie Rindern, Schafen, Ziegen, Katzen und Hunden, verwendet werden, können sie den Tieren sowohl als Einzeldosis wie auch wiederholt verabreicht werden, wobei die einzelnen Gaben je nach Tierart vorzugsweise zwischen 0,1 und 10 mg pro kg Körpergewicht betragen. Durch eine protrahierte Verabreichtung erzielt man in manchen Fällen eine bessere Wirkung oder man kann mit geringeren Gesamtdosen auskommen. Der Wirkstoff bzw. die ihn enthaltenden Mittel können auch dem Futter oder den Tränken zugesetzt werden. Das Fertigfutter enthält die Wirkstoffkombinationen vorzugsweise in einer Konzentration von 0,005 bis 0,1 Gew. %. Die Mittel können in Form von Lösungen, Emulsionen, Suspensionen, Pulver, Tabletten, Bolussen oder Kapseln peroral den Tieren verabreicht werden. Soweit die physikalischen und toxikologischen Eigenschaften von Lösungen oder Emulsionen dies zulassen, können die Wirkstoffe bzw. die sie enthaltenden Mittel den Tieren auch beispielsweise subcutan injiziert oder intraruminal verabreicht oder mittels der Pour-on-Methode auf den Körper der Tiere appliziert werden. Ferner ist eine Verabreichung des Wirkstoffs an die Tiere auch durch Lecksteine (Salz) oder Molasse-Blöcke möglich.

## Biologische Beispiele

### B-1. Insektizide Frassgift-Wirkung bei Spodoptera littoralis

Eingetopfte Baumwollpflanzen im 5-Blatt-Stadium werden mit einer acetonisch/wässrigen Versuchslösung besprüht, die 3, 12,5 oder 50 ppm der zu prüfenden Verbindung enthält.

Nach dem Antrocknen des Belags werden die Pflanzen mit ca. 30 Larven ($L_1$-Stadium) von Spodoptera littoralis besetzt. Pro Versuchsverbindung und pro Test-Spezies verwendet man zwei Pflanzen. Der Versuch wird bei ca. 24 °C und 60 % relativer Luftfeuchtigkeit durchgeführt. Auswertungen und Zwischenauswertungen auf moribunde Tiere, Wachstum der Larven und Frassschäden erfolgen nach 24 Stunden, 48 Stunden und 72 Stunden.

Verbindungen aus dem Umfang der Formeln I, und II (Tabelle 1 und 2), insbesondere die erfindungsgemäss neuen Oxime, zeigen bei einer Aufwandmenge von 12,5 ppm volle Wirksamkeit (100 %).

B-2. Wirkung gegen pflanzenschädigende Akariden

OP-sensible Tetranychus urticae

Die Primärblätter von Bohnenpflanzen (Phaseolus vulgaris) werden 16 Stunden vor dem Versuch mit einem durch T. urticae infestierten, aus einer Massenzucht stammenden Blattstück belegt. Die so mit allen Milben-Stadien infestierten Pflanzen werden nach Entfernung des Blattstücks mit einer Versuchslösung bis zur Tropfnässe besprüht, die 0,8 ppm der zu prüfenden Verbindung enthält. Die Temperatur in der Gewächshauskabine beträgt ca. 25 °C.

Nach sieben Tagen wird unter dem Binokular auf Prozentsatz mobiler Stadien (Adulte und Nymphen) und auf vorhandene Eier ausgewertet.

Verbindungen aus dem Umfang der Formel I und II (Tabelle 1 und 2), insbesondere auch die erfingungsgemäss neuen Oxime bewirken bei der verwendeten Wirkstoffkonzentration eine 60 %ige bis 100 %ige Abtötung.

B-3. Wirkung gegen $L_1$-Larven von Lucilia sericata

1 ml einer wässrigen Suspension der zu prüfenden Aktivsubstanz werden so mit 3 ml eines speziellen Larvenzuchtmediums bei ca. 50 °C vermischt, dass ein Homogenisat entsteht. In jede Reagenzglas-Probe werden ca. 30 Lucilia-Larven ($L_1$) eingesetzt. Nach 4 Tagen wird die Mortalitätsrate bestimmt. Verbindungen der Formeln I und II erzielen eine gute Wirkung gegenüber $L_1$-Larven von Lucilia sericata. Die erfindungsgemässen neuen Oxim-Verbindungen zeigen bereits in Aufwandmengen von 250 ppm volle Wirksamkeit (100 %).

B-4. Akarizide Wirkung gegen Boophilus microplus (Biarra-Stamm

Auf einer PVC-Platte wird waagrecht ein Klebstreifen so befestigt, das darauf 10 mit Blut vollgesogene Zecken-Weibchen von Boophilus microplus (Biarra-Stamm) nebeneinander in einer Reihe mit dem Rücken aufgeklebt werden können. Jeder Zecke wird mit einer Injectionsnadel 1 µl einer Flüssigkeit injiziert, die eine 1:1-Mischung von Polyethylenglykol und Aceton darstellt und in der eine bestimmte Wirkstoffmenge von wahlweise 1, 0,5 oder 0,1 µg pro Zecke gelöst ist. Kontrolltiere erhalten eine wirkstofffreie Injektion. Nach der Behandlung werden die Tiere unter Normalbedingungen in einem Insektarium bei ca. 28 °C und 80 % relativer Luftfeuchtigkeit gehalten, bis die Eiablage erfolgt und die Larven aus den Eiern der Kontrolltiere geschlüpft sind.

Die Aktivität einer geprüften Substanz wird mit der $IR_{90}$ bestimmt, d.h. es wird jene Wirkstoffdosis ermittelt, bei der noch nach 30 Tagen 9 von 10 Zeckenweibchen (= 90 %) Eier ablegen, die nicht schlupffähig sind.

Verbindungen der Formeln I und II (Tabelle 1 und 2) zeigen gute Aktivität gegen Boophilus microplus mit einem $IR_{90}$ von 1 µg. Die erfindungsgemässen neuen Oxim-Verbindungen erreichen bereits in sehr geringen Aufwandmengen (0,5 µg) ihren $IR_{90}$-Wert.

B-5. Versuch an mit Nematoden (Haemonchus concortus und Trichostrongylus colubriformis) infizierten Schafen

Der Wirkstoff wird als Suspension formuliert mit einer Magensonde oder durch Injektion in den Pansen eines Schafes gegeben, das mit Haemonchus concortus und Trychostrongylus colubriformis künstlich infiziert worden ist. Pro Dosis werden 1 bis 3 Tiere verwendet. Jedes Schaf wird nur einmal mit einer einzigen Dosis behandelt, und zwar wahlweise mit 1 mg oder 0,5 mg/kg Körpergewicht. Die Evaluierung erfolgt durch Vergleich der Anzahl der vor und nach Behandlung im Kot der Schafe ausgeschiedenen Wurmeier.

Gleichzeitig und gleichartig infizierte aber unbehandelte Schafe dienen als Kontrolle. Schafe, die mit einer der Verbindungen gemäss Tabellen 1 und 2 in einer Aufwandmenge von 1 mg/kg behandelt werden, zeigen im Vergleich zu unbehandelten, aber infizierten Vergleichsgruppen eine Reduktion des Nematodenbefalls im Bereich von 60 bis 100 % (100 % = komplette Reduktion der Wurmeier im Kot).

B-6. Kontaktwirkung auf Aphis craccivora

Erbsenkeimlinge, die mit sämtlichen Entwicklungsstadien der Laus infiziert sind, werden mit einer aus einem Emulsionskonzentrat hergestellten Wirkstofflösung besprüht, die wahlweise 50 ppm, 25 ppm oder 12,5 ppm Wirkstoff enthält. Nach 3 Tagen wird auf mehr als 80 % tote bzw. abgefallene Blattläuse ausgewertet. Nur bei dieser Wirkungshöhe wird ein Präparat als wirksam eingestuft.

Die Verbindungen der Formeln I und II aus den Tabellen, insbesondere auch die erfindungsgemässen neuen Oxim-Verbindungen erzielen bereits in geringen Konzentrationen zwischen 12,5 ppm und 25 ppm eine vollständige Abtötung (= 100 %).

B-7. Larvizidwirkung gegen Aedes aegypti

Auf die Oberfläche von 150 ml Wasser, das sich in einem Behälter befindet, wird soviel einer 0,1 %igen acetonischen Lösung des Wirkstoffes pipettiert, dass Konzentrationen von wahlweise 10 ppm, 3,3 ppm und 1,6 ppm erhalten werden. Nach Verdunsten des Acetons wird der Behälter mit ca. 30-40 3 Tage alten Aedes-Larven beschickt. Nach 1, 2 und 5 Tagen wird die Mortalität geprüft.

Verbindungen der Formeln I und II aus den Tabellen bewirkten in diesem Test bereits bei geringen Konzentrationen zwischen 3,3, und 10 ppm nach einem Tag eine vollständige Abtötung sämtlicher Larven.

B-8. Milbizide Wirkung gegen Dermanyssus gallinae

2 bis 3 ml einer Testlösung (100, 10, 1 und 0,1 ppm Aktivsubstanz) werden in einen nach oben offenen Glasbehälter gegeben und ca. 200 Milben in unterschiedlichen Entwicklungsstadien in diesen Behälter eingesetzt. Der Glasbehälter wird mit einem Wattebausch verschlossen und 10 Minuten gleichmässig, bis zur vollständigen Benetzung der Milben geschüttelt. Danach wird der Behälter umgekehrt hingestellt, bis die überschüssige Testlösung von der Watte aufgesogen ist. Der Behälter wird erneut gekehrt und die behandelten Milben zur Evaluierung der Wirksamkeit der Testsubstanzen drei Tage unter Laborbedingungen beobachtet, wobei die Mortalität als Massstab für Wirksamkeit herangezogen wird.

Verbindungen aus dem Umfang der Formeln I und II zeigen bei 100 ppm eine 100 %ige Wirksamkeit.

Literatur

Angelino S A G F, Müller F, Plas H C van der (1985) Purification and immobilization of rabbit liver aldehyde oxidase. Biotechnol Bioeng 27: 447-455

Bachmann S., Gebicka L, Gasyna Z (1981) Immobilisation of glucose isomerase on radiation-modified gelatine gel. Starch/Stärke 33: 63-66

Barbaric S, Kozulic B, Leustek I, Pavlovic B, Cesi V, Mildner P (1984) Crosslinking of glycoenzymes via their carbohydrate chains. In: 3rd Eur Congr Biotechnol, Bd 1. Verlag Chemie, Weinheim, S 307-312

Beddows C G, Guthrie J T, Abdel-Hay F I (1981) The use of graft copolymers as enzyme supports immobilization of proteins and enzymes on a hydrolyzed nylon-coacrylonitrile system. Biotechnol Bioeng 23: 2885-2889

Bettmann H, Rehm H J (1984) Degradation of phenol by polymer entrapped microorganisms. Appl Microbiol Biotechnol 20: 285-290

Bihari V, Goswami P P, Rizvi S H M, Kahn A W, Basu S K, Vora V C (1984) Studies on immobilized fungal spores of microbial transformation of steroids: 11a-hydroxylation of progesterone with immobilized spores of Aspergillus ochraceus G8 on polyacrylamide gel and other matrices. Biotechnol Bioeng 26: 1403-1408

Black G M, Webb C, Matthews T M, Atkinson B (1984) Practical reactor systems for yeast cell immobilization using biomass support particles. Biotechnol Bioeng 26: 134-141

Cabral J M S, Novais J M, Cardoso J P (1984) Coupling of glucoamylase on alkylamine derivative of titanium(IV) activated controlled pore glass with tannic acid. Biotechnol Bioeng 26: 386-388

Cannon J J, Chen L-F, Flickinger M C, Tsao G T (1984) The development of an immobilized lactate oxidase system for lactic acid analysis. Biotechnol Bioeng 26: 167-173

Cantarella M, Migliaresi C, Tafuri M G, Afani F (1984) Immobilization of yeast cells in hydroxymethacrylate gels. Appl Microbiol Biotechnol 20: 233-237

Chipley J R (1974) Effects of 2,4-dinitrophenol and N,N′-dicyclohexylcarbodiimide on cell envelopeassociated enzymes of Escherichia coli and Salmonella enteritidis. Microbios 10: 115-120

Clark-Still W, Kahn M, Mitra A (1978) J.Org.Chem. 43: 2923
Creuger W, Creuger A (1984) Biotechnologie-Lehrbuch der angewandten Mikrobiologie, 2. Auflage, R. Oldenbourg Verlag München, Wien, 1984.
Deo Y M, Gaucher G M (1984) Semicontinuous and continuous production of penicillin-G by Penicillium chrysogenum cells immobilized in k-carrageenan beads. Biotechnol Bioeng 26: 285-295
De Rosa M, Gambacorta A, Lama L, Nicolaus B (1981) Immobilization of thermophilic microbial cells in crude egg white. Biotechnol Lett 3: 183-188
DiLuccio R C, Kirwan D J (1984) Effect of dissolved oxygen on nitrogen fixation by A. vinelandii. II. Ionically adsorbed cells. Biotechnol Bioeng 26: 87-91
Erhardt H M, Rehm H J (1985) Phenol degradation by microorganisms adsorbed on activated carbon. Appl Microbiol Biotechnol 21: 32-35
Eikmeier H, Rehm H J (1984) Production of citric acid with immobilized Aspergillus niger. Appl Microbiol Biotechnol 20: 365-370
Förberg C, Häggström L (1984) Adsorbed cell systems controlled by the nutrient dosing technique. In: 3rd Eur Congr Biotechnol. Bd 2. Verlag Chemie, Weinheim, S 115-120
Gainer J L, Kirwan D J, Foster J A, Seylan E (1980) Use of adsorbed and covalently bound microbes in reactors. Biotechnol Bioeng Symp 10: 35-42
Giard D J, Loeb D H, Thilly W G, Wang D I C, Levine D W (1979) Human interferon production with diploid fibroblast cells grown on microcarriers. Biotechnol Bioeng 21: 433-443
Hartmeier W (1986), Immobilisierte Biokatalysatoren, Springer Verlag, Berlin, Heidelberg, New York, Tokyo, 1986.
Hofstee B H J (1973) Immobilization of enzymes through non-covalent binding to substituted agaroses. Biochem Biophys Res Commun 53: 1137-1144
Ibrahim M, Hubert P, Dellacherie E, Magadalou J, Muller J, Siest G (1985) Covalent attachment of epoxide hydrolase to dextran. Enz Microbiol Technol 7: 66-72
Jack T R, Zajic J E (1977) The enzymatic conversion of L-histidine to urocanic acid by whole cells of Micrococcus luteus immobilized on carbodiimide activated carboxymethylcellulose. Biotechnol Bioeng 19: 631
Jirku V, Turkova J, Krumphanzl V (1980) Immobilization of yeast with retention of cell division and extracellular production of macromolecules. Biotechnol Lett 2: 509-513
Karube I, Aizawa K, Ikeda S, Suzuki S (1979) Carbon dioxide fixation by immobilized chloroplasts. Biotechnol Bioeng 21: 253-260
Kato T, Horikoshi K (1984) Immobilized cyclomaltodextrin glucanotransferase of an alkalophilic Bacillus sp no 38-2. Biotechnol Bioeng 26: 595-598
Kaul R, D'Souza S F, Nadkarni G B (1984) Hydrolysis of milk lactose by immobilized $\beta$-galactosidase-hen egg white powder. Biotechnol Bioeng 26: 901-904
Khan S S, Siddiqi A M (1984) Studies on chemically aggregated pepsin using glutaraldehyde. Biotechnol Bioeng 27: 415-419
Krakowiak W, Jach M, Korona J, Sugier H (1984) Immobilization of glucoamylase on activated aluminiumoxide. Starch/Stärke 36: 396-398
Kühn W, Kirstein D, Mohr P (1980) Darstellung und Eigenschaften trägerfixierter Glukoseoxydase. Acta Biol med Germ 39: 1121-1128
Mazumder T K, Sonomoto K, Tanaka A, Fukui S (1985) Sequential conversion of cortexolone to prednisolone by immobilized mycelia of Curvularia lunata and immobilized cells of Arthrobacter simplex. App Microbiol Biotechnol 21: 154-161
Messing R A, Oppermann R A (1979) Pore dimensions for accumulating biomass. I. Microbes that reproduce by fission or budding. Biotechnol Bioeng 21: 49-58
Miyawaki O, Wingard jr L B (1984) Electrochemical and enzymatic activity of flavin dinucleotide and glucose oxidase immobilized by adsorption on carbon Biotechnol Bioeng 26: 1364-1371
Monsan P, Combes D, Alemzadeh I (1984) Invertase covalent grafting onto corn stover. Biotechnol Bioeng 26: 658-664
Mori T, Sato T, Tosa T, Chibata I (1972) Studies on immobilized enzymes. X. Preparation and properties of aminoacylase entrapped into acrylamide gel-lattice. Enzymologia 43: 213-226
Nakajima H, Sonomoto K, Usui N, Sato F, Yamada Y, Tanaka A, Fukui S (1985) Entrapment of Lavendula vera and production of pigments by entrapped cells. J. Biotechnol 2: 107-117
Navarro J M, Durand G (1977) Modification of yeast metabolism by immobilization onto porous glass. Eur J Appl Microbiol Biotechnol 4: 243-254
Präve P, Faust U. Sittig W, Sukatsch D A (1984) Handbuch Biotechnologie, 2. Auflage, R. Oldenbourg

Verlag München, Wien, 1984.
Qureshi N, Tamhane D V (1985) Production of mead by immobilized whole cells of Saccharomyces cerevisiae. Appl Microbiol Biotechnol 21: 280-281
Raghunath K, Rao K P, Joseph U T (1984) Preparation and characterization of urease immobilized onto collagen-poly(glycidyl methacrylate) graft copolymer. Biotechnol Bioeng 26: 104-109
Romanovskaya V A, Karpenko V I, Pantskhava E S, Greenberg T A, Malashenkp Y R (1981) Catalytic properties of immobilized cells of methane-oxidizing and methanogenic bacteria. In: Moo-Young M (Hrsg) Advances in Biotechnology, Bd 3. Pergamon, Toronto, S 367-372
Shimizu S, Morioka H, Tani Y, Ogata K (1975) Synthesis of coenzyme A by immobilized microbial cells. J. Ferm Technol 53: 77-83
Talsky G, Gianitsopoulos G (1984) Intermolecular crosslinking of enzymes. In: 3rd Eur Congr Biotechnol, Bd 1. Verlag Chemie, Weinheim, S 299-305
Umemura I, Takamatsu S, Sato T, Tosa T, Chibata I (1984) Improvement of production of L-aspartic acid using immobilized microbial cells. Appl Microbiol Biotechnol 20: 291-295
Van Haecht J L, Bolipombo M, Rouxhet P G (1985) Immobilization of Saccaromyces cerevisiae by adhesion: treatment of the cells by Al ions. Biotechnol Bioeng 27: 217-224
Vogel H J, Brodelius P (1984) an in vivo $_{31}$P NMR comparison of freely suspended and immobilized Catharanthus roseus plant cells. J Biotechnol 1: 159-170
Weetall H H, Mason R D (1973) Studies on immobilized papain. Biotechnol Bioeng 15: 455-466
Wiegel J, Dykstra M (1984) Clostridium thermocellum: adhesion and sporulation while adhered to cellulose and hemicellulose. Appl Microbiol Biotechnol 20: 59-65
Workman W E, Day D F (1984) Enzymatic hydrolysis of inulin to fructose by glutaraldehyde fixed yeast cells. Biotechnol Bioeng 26: 905-910.

Zitierte Patentliteratur:

US-PS 4,226,941
EP 0,147,852
EP 0,184,989
EP 0,184,173
US-PS 3,950,360
US-PS 4,346,171
US-PS 4,547,520
US-PS 4,173,571
DE-OS 35 32 794
US-PS 4,328,335

**Patentansprüche**

**1.** Einstufiges, mikrobielles Verfahren zur Herstellung von 13β-Hydroxi- oder 14,15-Epoxi-Milbemycinen der nachfolgenden Formeln I und II

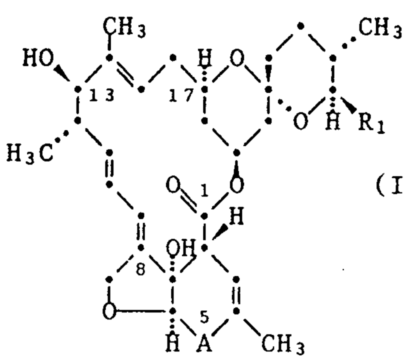
(I);

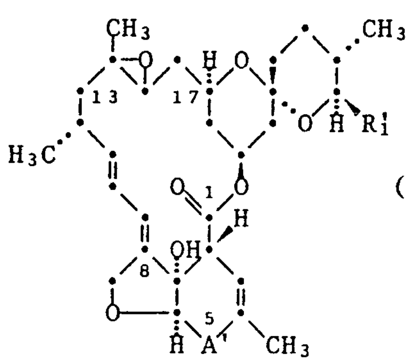
(II)

worin
$R_1$ und $R_1'$ für Methyl, Ethyl, Isopropyl oder sek.-Butyl oder für die Gruppe

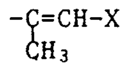

stehen, worin X Methyl, Ethyl oder Isopropyl bedeutet und A und A' für die Gruppe

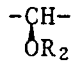 oder 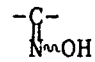

stehen, worin

$R_2$ Wasserstoff, -C(O)-$CH_2$O-C(O)-$CH_2CH_2$-COOH oder -C(O)-$CH_2CH_2$-COOH bedeutet, oder von Gemischen von Verbindungen der Formeln I und II, das sich dadurch kennzeichnet, dass man ein in einer flüssigen Phase gelöstes Milbemycin der Fomel III

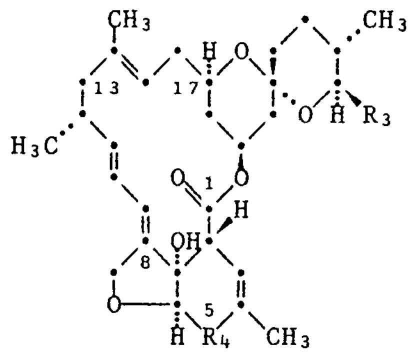

(III)

worin $R_3$ für $R_1$ oder $R_1'$ und $R_4$ für A oder A' steht und $R_1$, $R_1'$ , A und A' die unter den Formeln I und II angegebenen Bedeutungen haben mit einem zur 13$\beta$-Hydroxilierung oder 14,15-Epoxidierung oder zu beiden Reaktionen befähigten Biokatalysator für einen Zeitraum in Kontakt bringt, der für die Durchführung der 13$\beta$-Hydroxilierungs- oder 14,15-Epoxidierungsreaktion oder beider Reaktionen ausreicht.

**2.** Einstufiges, mikrobielles Verfahren gemäss Anspruch 1 zur Herstellung von 13$\beta$-Hydroxi oder 14,15-Epoxi-Milbemycinen der nachfolgenden Formeln I und II

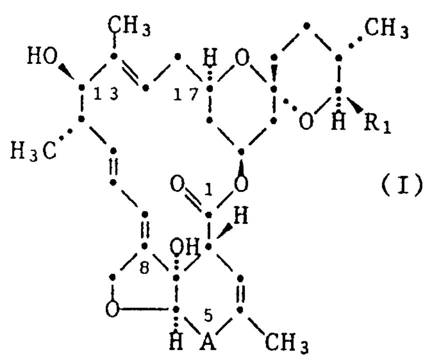

(I);

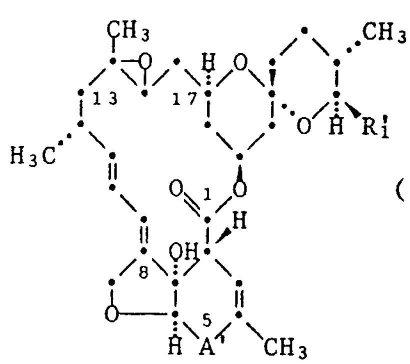

(II)

worin

$R_1$ und $R_1'$ für Methyl, Ethyl, Isopropyl oder sek.-Butyl und

A und A' für die Gruppe

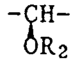 oder 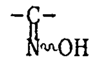

steht, worin

$R_2$ Wasserstoff, $-C(O)-CH_2O-C(O)-CH_2CH_2-COOH$ oder $-C(O)-CH_2CH_2-COOH$ bedeutet, dadurch gekennzeichnet, dass

man ein in einer flüssigen Phase gelöstes Milbemycin der Formel III,

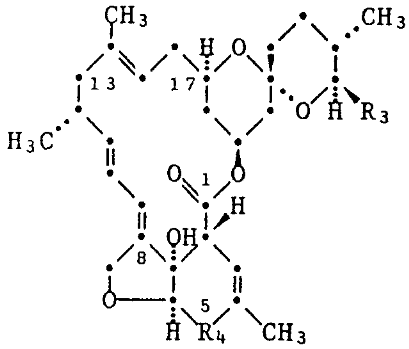

(III)

worin

$R_3$ für Methyl, Ethyl, Isopropyl oder sek.-Butyl und

$R_4$ für die Gruppe

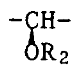 oder 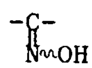

steht, worin

$R_2$ Wasserstoff, $-C(O)-CH_2O-C(O)-CH_2CH_2-COOH$ oder $-C(O)-CH_2CH_2-COOH$ bedeutet mit einem zur 13$\beta$-Hydroxilierung oder 14,15-Epoxidierung oder zu beiden Reaktionen befähigten Biokatalysator für einen Zeitraum in Kontakt bringt, der für die Durchführung der 13$\beta$-Hydroxilierungs-oder 14,15-Epoxidierungsreaktion oder beider Reaktionen ausreicht.

3. Verfahren gemäss Anspruch 1, dadurch gekennzeichnet, dass es sich bei besagtem Biokatalysator um einen Mikroorganismus handelt.

4. Verfahren gemäss Anspruch 3, dadurch gekennzeichnet, dass es sich bei besagtem Mikroorganismus um einen Vertreter aus der Gattung Streptomyces handelt.

5. Verfahren gemäss Anspruch 4, dadurch gekennzeichnet, dass es sich bei besagtem Vertreter um Streptomyces diastatochromogenes handelt.

6. Verfahren gemäss Anspruch 5, dadurch gekennzeichnet, dass es sich um Streptomyces diastatochromogenes ATCC 31561 handelt.

7. Verfahren gemäss Anspruch 4, dadurch gekennzeichnet, dass es sich bei besagtem Vertreter um Streptomyces violascens handelt.

8. Verfahren gemäss Anspruch 7, dadurch gekennzeichnet, dass es sich um Streptomyces violascens ATCC 31560 handelt.

9. Verfahren gemäss Anspruch 3, dadurch gekennzeichnet, dass es sich bei besagtem Mikroorganismus um einen Vertreter aus der Gattung Aspergillus handelt.

**10.** Verfahren gemäss Anspruch 9, dadurch gekennzeichnet, dass es sich bei besagtem Vertreter um Aspergillus niger handelt.

**11.** Verfahren gemäss Anspruch 10, dadurch gekennzeichnet, dass es sich um Aspergillus niger ATCC 20567 handelt.

**12.** Verfahren gemäss Anspruch 1, dadurch gekennzeichnet, dass es sich bei besagtem Biokatalysator um einen toten Mikroorganismus in einer aufgeschlossenen Form handelt.

**13.** Verfahren gemäss Anspruch 1, dadurch gekennzeichnet, dass es sich bei besagtem Biokatalysator um einen Rohextrakt der Zellinhaltsstoffe handelt.

**14.** Verfahren gemäss Anspruch 1, dadurch gekennzeichnet, dass es sich bei besagten Biokatalysator um ein Enzym handelt.

**15.** Verfahren gemäss Anspruch 1, dadurch gekennzeichnet, dass es sich bei besagten Biokatalysator um Sporen handelt.

**16.** Verfahren nach einem der Ansprüche 1 bis 14, dadurch gekennzeichnet, dass besagter Biokatalysator in immobilisierter Form vorliegt.

**17.** Verfahren gemäss Anspruch 1, dadurch gekennzeichnet, dass die Hydroxilierungs- oder Epoxidierungsreaktion oder beide Reaktionen in einem Bioreaktor durchgeführt werden.

**18.** Verfahren gemäss Anspruch 17, dadurch gekennzeichnet, dass es sich bei besagtem Bioreaktor um einen Rührreaktor handelt.

**19.** Verfahren gemäss Anspruch 1, dadurch gekennzeichnet, dass man
a) besagten Mikroorganismus in einem komplexen Nährmedium, enthaltend eine wenig definierte, leicht assimilierbare Kohlenstoff- und Stickstoffquelle sowie als weitere essentielle Bestandteile Vitamine, Metallionen und Spurenelemente, kultiviert,
b) nach einem angemessenen Zeitraum, der eine für die Hydroxilierungs- oder Epoxidierungsreaktion oder für beide Reaktionen optimale Zelldichte garantiert, eine Verbindung der Formel III zugibt,
c) den Biokatalysator und sein Substrat (Verbindungen der Formel III) für einen Zeitraum, der für die Durchführung der Hydroxilierungs- oder Epoxidierungsreaktion oder für die Durchführung beider Reaktionen ausreicht, miteinander inkubiert, und
d) das Reaktionsprodukt mit Hilfe an sich bekannter Verfahren isoliert.

**20.** Verfahren gemäss Anspruch 19, dadurch gekennzeichnet, dass die Prozesstemperatur bei Werten zwischen 20 °C und 32 °C liegt.

**21.** Verfahren gemäss Anspruch 19, dadurch gekennzeichnet, dass der pH-Wert des Kulturmediums zwischen pH 4 und pH 8 liegt.

**22.** Verfahren gemäss Anspruch 19, dadurch gekennzeichnet, dass besagte Komplexe Nährmedien
a) als Stickstoffquelle Hefe-Extrakt, Hefe-Hydrolysat, Hefe-Autolysat, Hefe-Zellen, Sojamehl, Maismehl, Hafermehl, Edamin, Pepton, Caseinhydrolysat, Maisablaugen, Fleischextrakt oder jede beliebige andere leicht assimilierbare N-Quelle,
b) als Kohlenstoffquelle Glucose, Lactose, Sucrose, Dextrose, Maltose, Stärke, Cerelose, Cellulose, Malzextrakt oder jede andere leicht assimilierbare C-Quelle
c) als Vitamine Thiamin, Biotin, Nicotinsäure, Pyridoxalphosphat und andere essentielle Vitamine
d) als anorganische Metallionen $Na^+$, $K^+$, $Ca^{2+}$, $Mg^{2+}$, $NH_4^{\oplus}$, $PO_4^{3-}$, $SO_4^{2-}$, $Cl^-$, $CO_3^{2-}$ und andere essentielle Metallionen in Form ihrer Salze
e) sowie als Spurenelemente $Mn^{2+}$, $Co^{2+}$, $Zn^{2+}$ und andere essentielle Spurenelemente in Form ihrer Salze

in einer Konzentration enthalten, einzeln oder in Kombination, die für ein optimales Zell-Wachstum sowie die Bereitstellung optimaler Reaktionsbedingungen notwendig ist.

**23.** Verfahren gemäss Anspruch 18, dadurch gekennzeichnet, dass die Konzentration der Stickstoffquelle in einem Bereich zwischen 0,1 g/l und 6 g/l liegt.

**24.** Verfahren gemäss Anspruch 18, dadurch gekennzeichnet, dass die Konzentration der Kohlenstoffquelle in einem Bereich zwischen 1,0 g/l und 25 g/l liegt.

## Claims

**1.** A one-step microbial process for the preparation of 13β-hydroxy- or 14,15-epoxy-milbemycins of the following formulae I and II

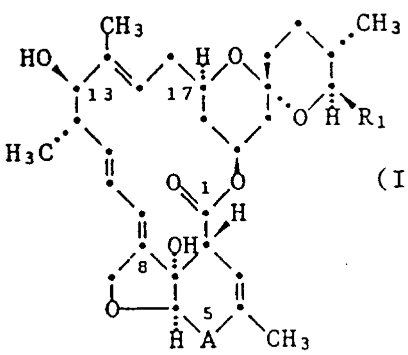

(I);

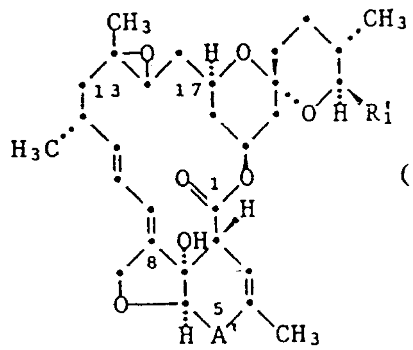

(II)

in which
$R_1$ and $R_1'$ represent methyl, ethyl, isopropyl or sec.-butyl or represent the group

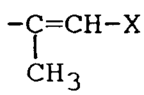

in which X represents methyl, ethyl or isopropyl, and
A and A' represent the group

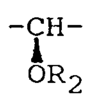

or

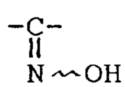

in which
$R_2$ represents hydrogen, $-C(O)-CH_2O-C(O)-CH_2CH_2-COOH$
or $-C(O)-CH_2CH_2-COOH$, or mixtures of compounds of the formulae I and II, which process comprises bringing a milbemycin of the formula III

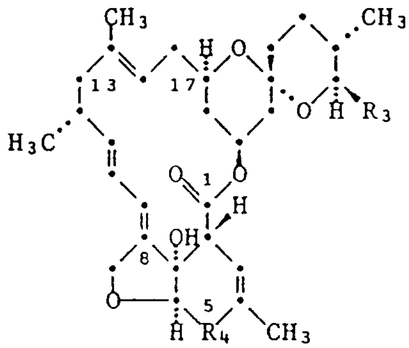

(III)

dissolved in a liquid phase, in which $R_3$ represents $R_1$ or $R_1'$ and $R_4$ represents A or A', and $R_1$, $R_1'$ , A and A' have the meanings given for formulae I and II, into contact with a biocatalyst that is capable of 13β-hydroxylation or 14,15-epoxidation, or of both reactions, for a period of time that is sufficient for carrying out the 13β-hydroxylation or 14,15-epoxidation reaction or both reactions.

**2.** A one-step microbial process according to claim 1 for the preparation of 13β-hydroxy- or 14,15-epoxy-milbemycins of the following formulae I and II

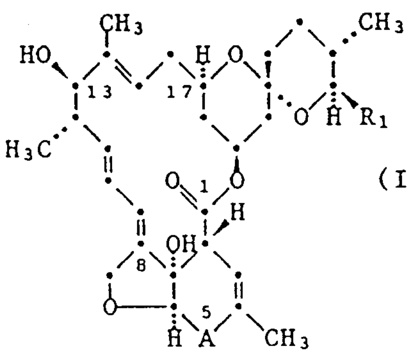

(I);

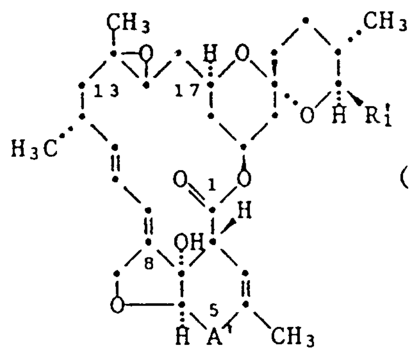

(II)

in which
$R_1$ and $R_1'$ represent methyl, ethyl, isopropyl or sec.-butyl and
A and A' represent the group

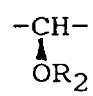

or

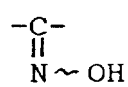

in which
$R_2$ represents hydrogen, $-C(O)-CH_2O-C(O)-CH_2CH_2-COOH$ or $-C(O)-CH_2CH_2-COOH$,
which process comprises bringing a milbemycin of the formula III

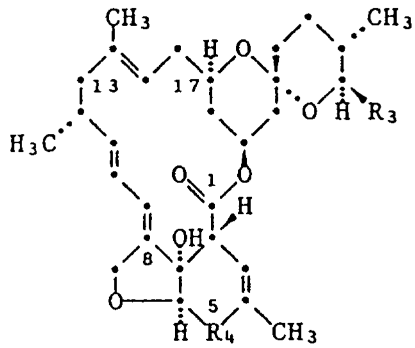

dissolved in a liquid phase, in which
$R_3$ represents methyl, ethyl, isopropyl or sec.-butyl and
$R_4$ represents the group

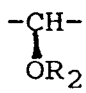
or
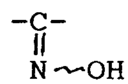

in which
$R_2$ represents hydrogen, -C(O)-$CH_2$O-C(O)-$CH_2CH_2$-COOH or -C(O)-$CH_2CH_2$-COOH into contact with a biocatalyst that is capable of 13β-hydroxylation or 14,15-epoxidation, or of both reactions, for a period of time that is sufficient for carrying out the 13β-hydroxylation or 14,15-epoxidation reaction or both reactions.

**3.** A process according to claim 1 wherein the said biocatalyst is a microorganism.

**4.** A process according to claim 3 wherein the said microorganism is a representative from the Streptomyces species.

**5.** A process according to claim 4 wherein the said representative is Streptomyces diastatochromogenes.

**6.** A process according to claim 5 wherein the said representative is Streptomyces diastatochromogenes ATCC 31561.

**7.** A process according to claim 4 wherein the said representative is Streptomyces violascens.

**8.** A process according to claim 7 wherein the said representative is Streptomyces violascens ATCC 31560.

**9.** A process according to claim 3 wherein the said microorganism is a representative from the Aspergillus species.

**10.** A process according to claim 9 wherein the said representative is Aspergillus niger.

**11.** A process according to claim 10 wherein the said representative is Aspergillus niger ATCC 20567.

**12.** A process according to claim 1 wherein the said biocatalyst is a dead microorganism in an opened-up form.

**13.** A process according to claim 1 wherein the said biocatalyst is a crude extract of the cell contents.

**14.** A process according to claim 1 wherein the said biocatalyst is an enzyme.

**15.** A process according to claim 1 wherein the said biocatalyst is spores.

**16.** A process according to any one of claims 1 to 14 wherein the said biocatalyst is in immobilised form.

**17.** A process according to claim 1 wherein the hydroxylation or epoxidation reaction or both reactions are carried out in a bioreactor.

**18.** A process according to claim 17 wherein the said bioreactor is a stirred vessel reactor.

**19.** A process according to claim 1 which comprises

a) culturing the said microorganism in a complex nutrient medium comprising a poorly defined, readily assimilable carbon source and nitrogen source and, as other essential constituents, vitamins, metal ions and trace elements,

b) after an appropriate period of time that guarantees a cell density that is optimum for the hydroxylation or epoxidation reaction or for both reactions, adding a compound of the formula III,

c) incubating the biocatalyst and its substrate (compounds of the formula III) together for a period of time that is sufficient for carrying out the hydroxylation or epoxidation reaction or both reactions, and

d) isolating the reaction product by processes that are known per se.

**20.** A process according to claim 19 wherein the process temperature is from 20 °C to 32 °C.

**21.** A process according to claim 19 wherein the pH value of the culture medium is from pH 4 to pH 8.

**22.** A process according to claim 19 wherein the said complex nutrient media comprise

a) as nitrogen source: yeast extract, yeast hydrolysate, yeast autolysate, yeast cells, soya meal, maize meal, oatmeal, edamine, peptone, casein hydrolysate, corn steep liquors, meat extract or any other readily assimilable nitrogen source,

b) as carbon source: glucose, lactose, sucrose, dextrose, maltose, starch, cerelose, cellulose, malt extract or any other readily assimilable carbon source,

c) as vitamins: thiamin, biotin, nicotinic acid, pyridoxalphosphate and other essential vitamins,

d) as inorganic metal ions: $Na^+$, $K^+$, $Ca^{2+}$, $Mg^{2+}$, $NH_4^{\oplus}$,$PO_4^{3-}$, $SO_4^{2-}$, $Cl^-$, $CO_3^{2-}$ and other essential metal ions in the form of their salts,

e) and as trace elements: $Mn^{2+}$, $Co^{2+}$, $Zn^{2+}$ and other essential trace elements in the form of their salts,

individually or in combination, in a concentration necessary for optimum cell growth and for the provision of optimum reaction conditions.

**23.** A process according to claim 18 wherein the concentration of the nitrogen source is in a range of from 0.1 g/l to 6 g/l.

**24.** A process according to claim 18 wherein the concentration of the carbon source is in a range of from 1.0 g/l to 25 g/l.

## Revendications

**1.** Procédé microbien, en une étape, pour la préparation de la 13$\beta$ -hydroxy- ou 14,15-époxymilbémycine répondant aux formules I et II suivantes

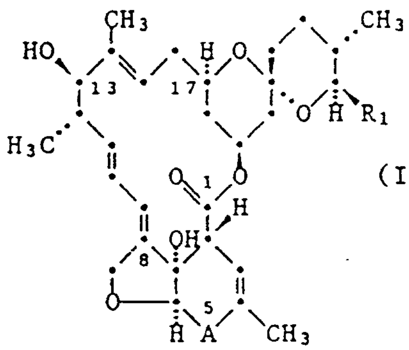

(I);

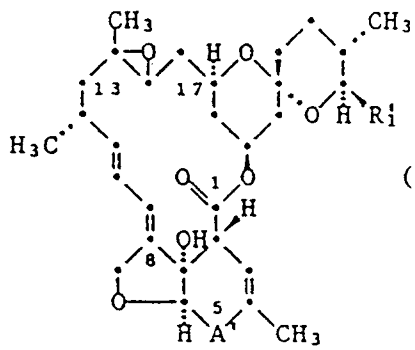

(II)

où

$R_1$ et $R_1'$ représentent le méthyle, l'éthyle, l'isopropyle ou le sec-butyle ou le groupe

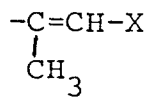

dans lequel X représente le méthyle, l'éthyle ou l'isopropyle et

A et A' représentent le groupe

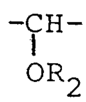

ou

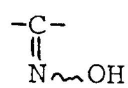

dans lequel

$R_2$ représente l'hydrogène, $-C(O)-CH_2O-C(O)-CH_2CH_2-COOH$

ou $-C(O)-CH_2CH_2-COOH$ ou des mélanges des composés de formules I et II,

caractérisé en ce que l'on met en contact de la milbémycine de formule III dissoute dans une phase liquide

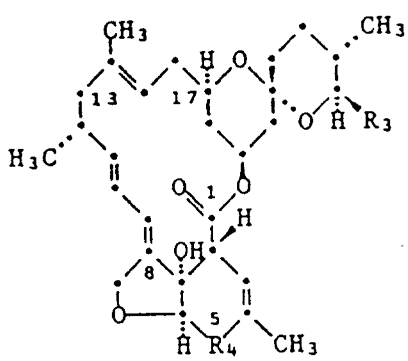

(III)

où $R_3$ représente $R_1$ ou $R_1'$ et $R_4$ représente A ou A' et $R_1$, $R_1'$ , A et A' ont les significations données pour les formules I et II, avec un biocatalyseur capable d'effectuer une hydroxylation en 13$\beta$ ou une époxydation en 14,15 ou cas deux réactions pendant un laps de temps suffisant pour réaliser la réaction d'hydroxylation en 13$\beta$ ou la réaction d'époxydation en 14,15 ou ces deux réactions.

2. Procédé microbien, en une étape, selon la revendication 1, pour la préparation de la 13$\beta$ -hydroxyou de la 14,15-époxymilbémycine répondant aux formules I et II suivantes

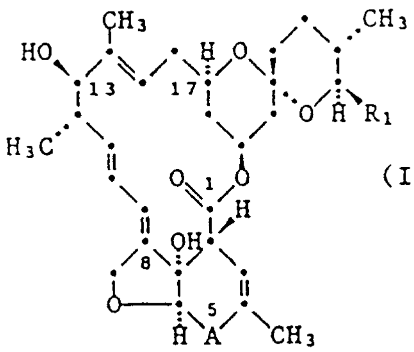
(I);

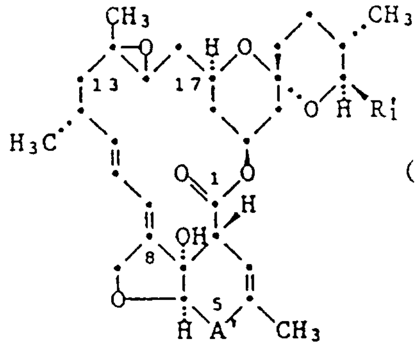
(II)

où
$R_1$ et $R_1'$ représentent le méthyle, l'éthyle, l'isopropyie ou le sec-butyle et
A et A' représentent le groupe

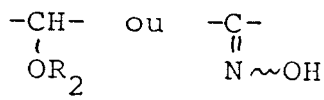

dans lequel R représente l'hydrogène, $-C(O)-CH_2O-C(O)-CH_2CH_2-COOH$ ou $-C(O)-CH_2CH_2-COOH$, caractérisé en ce que l'on met en contact la milbémycine de formule III dissoute dans une phase liquide

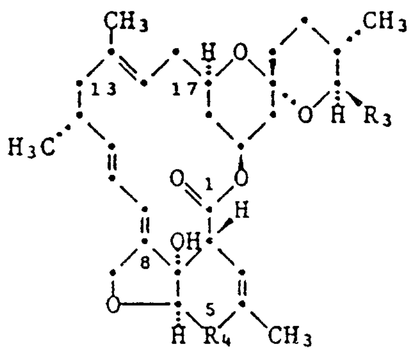
(III)

où
$R_3$ représente le méthyle, l'éthyle, l'isopropyle ou le sec-butyle et
$R_4$ représente le groupe

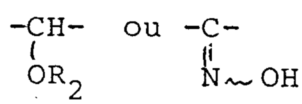

dans lequel
$R_2$ représente l'hydrogène, $-C(O)-CH_2O-C(O)-CH_2CH_2-COOH$
ou $-C(O)-CH_2CH_2-COOH$ avec un biocatalyseur capable d'effectuer une hydroxylation en 13$\beta$ ou une époxydation en 14,15 ou ces deux réactions, pendant un laps de temps suffisant pour réaliser la réaction d'hydroxylation en 13$\beta$ ou la réaction d'époxydation en 14,15 ou ces deux réactions.

**3.** Procédé selon la revendication 1, caractérisé en ce que ledit biocatalyseur est un microorganisme.

**4.** Procédé selon la revendication 3, caractérisé en ce que ledit microorganisme est un représentant du genre Streptomyces.

**5.** Procédé selon la revendication 4, caractérisé en ce que ledit représentant est Streptomyces diastatochromogenes.

**6.** Procédé selon la revendication 5, caractérisé en ce qu'il s'agit de Streptomyces diastatochromogenes ATCC 31 561.

**7.** Procédé selon la revendication 4, caractérisé en ce que ledit représentant est Streptomyces violascens.

**8.** Procédé selon la revendication 7, caractérisé en ce qu'il s'agit de Streptomyces violascens ATCC 31 560.

**9.** Procédé selon la revendication 3, caractérisé en ce que ledit microorganisme est un représentant du genre Aspergillus.

**10.** Procédé selon la revendication 9, caractérisé en ce que ledit représentant est Aspergillus niger.

**11.** Procédé selon la revendication 10, caractérisé en ce qu'il s'agit d'Aspergillus niger ATCC 20 567.

**12.** Procédé selon la revendication 1, caractérisé en ce que ledit biocatalyseur est un microorganisme mort sous forme désagrégée.

**13.** Procédé selon la revendication 1, caractérisé en ce que ledit biocatalyseur est un extrait brut des constituants cellulaires.

**14.** Procédé selon la revendication 1, caractérisé en ce que ledit biocatalyseur est un enzyme

**15.** Procédé selon la revendication 1, caractérisé en ce que ledit biocatalyseur est constitué par des spores.

**16.** Procédé selon l'une des revendications 1 à 14, caractérisé en ce que ledit biocatalyseur se présente sous forme immobilisée.

**17.** Procédé selon la revendication 1, caractérisé en ce que la réaction d'hydroxylation ou d'époxydation ou les deux réactions sont effectuées dans un bioréacteur.

**18.** Procédé selon la revendication 17, caractérisé en ce que ledit bioréacteur est un réacteur à agitation.

**19.** Procédé selon la revendication 1, caractérisé

a) en ce que l'on utilise ledit microorganisme dans un milieu nutritif complexe, contenant une source de carbone et une source d'azote, peu définies, facilement assimilables, ainsi que comme autres constituants essentiels, des vitamines, des ions métalliques et des oligo-éléments,

b) en ce que l'on additionne, après un laps de temps raisonnable, un composé de formule III garantissant une densité cellulaire optimale pour la réaction d'hydroxylation ou la réaction d'époxydation ou pour les deux réactions,

c) en ce que l'on incube le biocatalyseur et son substrat (composés de formule III) pendant un laps de temps suffisant pour la réalisation de la réaction d'hydroxylation ou d'époxydation ou pour la réalisation des deux réactions et

d) en ce que l'on isole le produit de la réaction par un procédé connu en soi.

**20.** Procédé selon la revendication 19, caractérisé en ce que la température du procédé est comprise entre 20 ° C et 32 ° C.

**21.** Procédé selon la revendication 19, caractérisé en ce que le pH du milieu de culture est compris entre 4 et 8.

**22.** Procédé selon la revendication 19, caractérisé en ce que lesdits milieux nutritifs complexes contiennent

a) comme source d'azote, de l'extrait de levure, de l'hydrolysat de levure, de l'autolysat de levure, des cellules de levure, de la farine de soja, de la farine de maïs, de la farine d'avoine, de l'édamine, de la peptone, de l'hydrolysat de caséine, des eaux de lavage de maïs concentrées, de l'extrait de viande ou tout autre source de N facilement assimilable,

b) comme source de carbone, du glucose, du lactose, du saccharose, du dextrose, du maltose, de l'amidon, du cerelose, de la cellulose, de l'extrait de malt ou tout autre source de C facilement assimilable,

c) comme vitamines, de la thiamine, de la biotine, de l'acide nicotinique, du phosphate de pyridoxal et d'autres vitamines essentielles,

d) comme ions métalliques minéraux $Na^+$, $K^+$, $Ca^{2+}$, $Mg^{2+}$, $NH_4^{\oplus}$, $MO_4^{3-}$, $SO_4^{2-}$, $Cl^-$, $CO_3^{2-}$ et d'autres ions métalliques essentiels sous forme de leurs sels,

e) ainsi que comme oligo-éléments $Mn^{2+}$, $Co^{2+}$, $Zn^{2+}$ et d'autres oligo-éléments essentiels sous forme de leurs sels,

seul ou en combinaison, à la concentration requise pour une croissance optimale des cellules, ainsi que pour l'établissement de conditions réactionnelles optimales.

**23.** Procédé selon la revendication 18, caractérisé en ce que la concentration de la source d'azote est comprise entre 0,1 g/l et 6 g/l.

**24.** Procédé selon la revendication 18, caractérisé en ce que la concentration de la source de carbone est comprise entre 1,0 g/l et 25 g/l.